(19) Europäisches Patentamt
European Patent Office
Office européen des brevets

(11) Publication number: **0 148 155 B1**

(12) **EUROPEAN PATENT SPECIFICATION**

(45) Date of publication of patent specification: **19.04.89**

(51) Int. Cl.⁴: **C 07 K 15/06**, C 07 K 15/12, A 61 K 35/32, A 61 K 37/02

(21) Application number: **85100047.1**

(22) Date of filing: **02.01.85**

(54) **Osteogenic factors.**

(30) Priority: **04.01.84 US 568167**

(43) Date of publication of application: **10.07.85 Bulletin 85/28**

(45) Publication of the grant of the patent: **19.04.89 Bulletin 89/16**

(84) Designated Contracting States: **AT BE CH DE FR GB IT LI LU NL SE**

(56) References cited:
**US-A-4 294 753**

**CHEMICAL ABSTRACTS, vol. 98, no. 11, 14th March 1983, page 200, no. 85039r, Columbus, Ohio, US; H. MIZUTANI et al.: "The nature of bone morphogenetic protein (BMP) fractions derived from bovine bone matrix gelatin", & CLIN. ORTHOP. RELAT. RES. 1982, 171, 213-23**
**PROC. NATL. ACAD. SCI. USA, vol. 81, January 1984, pages 371-375; M.R. URIST et al.: "Purification of bovine bone morphongenetic protein by hydroxyapatite chromatography"**

(73) Proprietor: **INTERNATIONAL GENETIC ENGINEERING, INC.**
**1545 - 17th Street**
**Santa Monica California 90404 (US)**

(72) Inventor: **Sen, Arup**
**11733 Kiowa Avenue 6th**
**Los Angeles California 90049 (US)**

(74) Representative: **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820**
**D-8000 München 86 (DE)**

(56) References cited:
**SCIENCE, vol. 220, no. 4598, 13th May 1983, pages 680-686; M.R. URIST et al.: "Bone cell differentiation and growth factors"**

**CHEMICAL ABSTRACTS, vol. 96, no. 19, 10th May 1982, page 298, no. 157675d, Columbus, Ohio, US; M.R. URIST et al.: "A bonvine low molecular weight bone morphogenetic protein (BNP) fraction", & CLIN. ORTHOP. RELAT. RES. 1982, 162, 219-32**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background

Bone is a highly specialized connective tissue with unique mechanical properties derived from its extensive matrix structure. A network of fibrous bundles composed of the protein collagen is presumed to provide the tension-resistant behavior of bone. In addition other materials including proteoglycans, noncollagenous proteins, lipids and acidic proteins associated with a mineral phase consisting primarily of poorly crystallized hydroxyapatite are deposited in the extensive matrix architecture of bone. Bone tissue is continuously renewed throughout the life of mammals. This physiologic process might serve to maintain the properties of a young tissue.

The processes of bone formation and renewal are carried out by specialized cells. Osteogenesis vis-a-vis morphogenesis and growth of bone is presumably carried out by the "osteoblasts" (bone-forming cells). Remodeling of bone is apparently brought about by an interplay between the activities of the bone-resorbing cells called "osteoclasts" and the bone-forming osteoblasts. The bony skeleton is thus not only an architectural structure with a mechanical function but also is a living tissue capable of growth, modeling, remodeling and repair. Since these processes are carried out by specialized living cells, chemical (pharmaceutical/hormonal), physical and physicochemical alterations can affect the quality, quantity and shaping of bone tissue.

A variety of pathological disorders as well as physical stress (for example, fracture) necessitate active formation of bone tissue at rates that are significantly higher than that which can be supported by the normal milieu of the body. It is thus of value to identify physiologically acceptable chemical agents (hormones/pharmaceuticals/growth factors) that can induce the formation of bone at a predetermined site. Such agents could either provide a permissive matrix structure for the deposition of bone-forming cells or cause growth stimulation of bone-forming cells or induce the differentiation of appropriate progenitors of bone-forming cells.

The presence of proteinaceous and prostaglandin-like growth stimulators for osteoblasts has been examined, see Raisz, L. G., et al., The New England Journal of Medicine, Vol. 309, No. 1, pp. 29—35 (1983) and Raisz, L. G., et al., The New England Journal of Medicine, Vol. 309, No. 2, pp. 83—89 (1983).

Urist et al. have been able to provide evidence that bone matrix-associated noncollagenous proteins can be isolated by dissociative treatment of demineralized bone powder and that this mixture of extracted materials as well as partially fractionated materials obtained therefrom contain bone morphogenetic activity, see Urist, M. R., et al., Proc. Natl. Acad. Sci. USA, Vol. 76, No. 4, pp. 1828—1832 (1979); Urist, M. R., et al., Proceedings of the Society of Experimental Biology and Medicine, Vol. 162, pp. 48—53 (1979); Hanamura, H., et al., Clinical Orthopaedics, Vol. 148, pp. 281—290 (1980); Urist, M. R., U.S. Patent No. 4,294,753 (1981); Urist, M. R., U.S. Patent No. 4,455,256 (1984); Urist, M. R., et al., Clinical Orthopaedics, Vol. 162, pp. 219—232 (1982); Urist, M. R., et al., Science, Vol. 220, pp. 680—686 (1983), and H. Mizutani and M. R. Urist Clin. Orthop. Rel. Res. 171 (1982) 213—223, ref. in Chemical Abstracts 98(1983) 85034r.

Baylink and his collaborators have been able to identify a separate type of activity which presumably couples bone resorption with new bone formation, see Howard, G. A., et al., Metabolic Bone Disease and Related Research, Vol. 2, pp. 131—135 (1980); Farley, J. R., et al., Biochemistry, Vol. 21, pp. 3502—3507 (1982) and Farley, J. R., et al., Biochemistry, Vol. 21, pp. 3508—3513 (1982). The activity which Farley et al. obtained from bone matrix involves a different extraction procedure than that of the present invention or that of Urist, supra, it has a larger molecular weight and was called "skeletal growth factor" or "skeletal coupling factor".

The procedures and techniques known in the art for obtaining putative osteogenic activities suffer from several flaws. The isolation procedures are prolonged, ill-defined and incomplete. As such, a definitive association of the activity with a chemically characterized, highly purified protein preparation has not been established. A protein of approximately 17,000 daltons obtained from calf bone powder has been termed "bone morphogenetic protein", see Urist, M. R., et al., Science, Vol. 220, pp. 680—686 (1983); it is claimed to induce efficient bone formation especially when present in a multimolecular assembly with certain other bone-derived proteins which in the absence of the 17,000 dalton protein are non-osteogenic. Urist et al., Proceedings of The Society of Experimental Biology and Medicine, Vol. 173, pp. 194—199 (1983), also identified a 17,000 to 18,000 dalton protein from human bone; this protein is claimed to induce efficient bone formation when administered together with 24,000 and 14,000 dalton human bone-derived proteins. The 24,000 and 14,000 dalton proteins are osteogenically inactive when used without the 17,000 to 18,000 dalton protein but might serve as carriers of the active 17,000 to 18,000 dalton human bone protein. Urist, M. R., et al., Proc. Natl. Acad. Sci. USA, Vol. 81, pp. 371—375 (January, 1984) identifies a 18,500±500 dalton protein isolated from bovine bone and referred to as bovine bone morphogenetic protein. The 18,500 dalton protein induced bone formation when implanted alone or with various combinations of other bone derived proteins. Only samples containing the 18,500 dalton protein induced bone formation. It was further indicated that other bovine bone derived proteins having molecular weights of 14,000, 17,000, 17,500, 22,000 or 34,000 daltons, respectively, when implanted alone or in various combinations failed to induce bone formation. A bovine bone derived protein of 24,000 daltons was also mentioned and there was no indication that the 24,000 dalton protein induced bone formation.

Less pure preparations containing proteins of molecular weights between 17,000 to 23,000 daltons and

claimed to possess bone-morphogenetic activities have been isolated from sources such as mouse osteosarcoma, see Hanamura, H., et al., Clinical Orthopaedics, Vol. 153, pp. 232—240 (1980), and rabbit dentin, see Conover, M. A., and Urist, M. R., The Chemistry and Biology of Mineralized Connective Tissues, Elsevier North Holland, Inc., pp. 597—606 (1981).

Protein preparations used in most of the osteogenic activity measurement experimentations described in the literature to date have been of insufficient purity and thus have not led to the identification of the specific molecular entities responsible for the observed activities. Furthermore, studies reported to date have failed to reveal any chemical (biochemical) relationship between the active protein species present in the various "bone morphogenetic protein" preparations.

For example, the teachings of U.S. Patent Nos. 4,294,753, 4,455,256 and 4,434,094 all concern processes for fractionation of crude protein mixtures obtained by dissociative extractions of demineralized bone matrix. The teachings of U.S. Patent Nos. 4,294,753, 4,455,256 and 4,434,094 at best yield a mixture of different proteins and not an essentially homogeneous protein species identified as having osteogenic activity.

## Summary of the invention

The present invention concerns a family of immunologically related primary osteogenic factors (also referred to herein as "P3 proteins"), each of which in its substantially pure state induces bone formation at a predetermined site in a mammal when applied alone or in admixture with a suitable pharmaceutically acceptable carrier.

Subject of the present invention is a preparation of an osteogenic protein promoting osteogenesis and a method of isolating the preparation according to claims 1 and 5. Preferred embodiments of these are set out in claims 2 to 4 and 6 to 8, respectively.

Using the procedures described herein, proteins (such proteins are further described herein and have been given identifying names such as P1, P2, P3, P4 and the like based on their migration in polyacrylamide gels under dissociating, reducing conditions) can be obtained from the bone of each mammalian species. Among the proteins which are so obtained, there is a protein referred to herein as the "primary osteogenic factor" (also referred to herein by its identifying name as the "P3 protein") which induces bone formation at a predetermined site in a mammal when administered alone or in admixture with a suitable pharmaceutically acceptable carrier. A particular primary osteogenic factor can be obtained from the bone of a particular mammalian species. For example, the primary osteogenic factor obtained from bovine bone corresponds to and is immunologically related to the primary osteogenic factor obtained from human bone, and corresponds to and is immunologically related to the respective primary osteogenic factors which can be isolated from the bone of other mammalian species. In this invention the term "primary osteogenic factor" or "P3 protein" refers to a particular P3 protein endogenous to the bone of a particular mammalian species. Thus this family of immunologically related proteins is comprised of the P3 proteins obtained from the bone of respective mammalian species, each P3 protein being a molecular species variant of the other members of the family.

The osteogenic substances of the present invention include the osteogenic factors selected from the group consisting of: (a) the proteins of the P3 family of immunologically related proteins (that is, the P3 proteins); (b) the osteogenically active polypeptides derived from said P3 proteins; (c) polypeptides which are or can be converted to osteogenically active entities which are immunologically related to one or more of the P3 proteins; and (d) mixtures of osteogenic factors selected from (a), (b) and (c).

In the present invention, the preferred P3 proteins are: the P3 protein which can be obtained from bovine bone (such as calf bone), and referred to herein either as "bovine P3 protein" or "calf P3 protein"; the P3 protein which can be obtained from human bone, referred to herein as "human P3 protein"; and the P3 protein which can be obtained from porcine bone, referred to herein as "porcine P3 protein".

The P3 proteins of the present invention exhibit the ability to promote or stimulate osteogenesis at desired locations in mammals. In the method of using the osteogenic factors of the present invention, it is preferred to use an osteogenic factor obtained from a particular mammalian species for administration to that mammalian species (for example, the P3 protein obtained from human bone is preferred for administration to humans); however, the use of P3 proteins obtained from the bone of mammalian species which are different from the mammalian species to be treated is within the scope of the present invention.

Using procedures well known in the art, for example, chemical, enzymatic or recombinant DNA techniques, it may be possible to obtain polypeptides derived from the osteogenic P3 proteins described herein which exhibit the ability to promote or stimulate osteogenesis. Proteins or polypeptides that are or can be converted to osteogenically active species which are immunologically related to the P3 proteins or fragments thereof are also considered to be within the scope of the present invention. Osteogenically active entities, referred to herein as "active polypeptides" or "osteogenically active polypeptides", include any portion of the proteins or polypeptides which are the subject of the present invention having osteogenic activity and functional derivatives thereof having osteogenic activity and includes any osteogenically active entities that can be produced by conventional procedures such as chemical synthesis, enzymatic modification or recombinant DNA techniques. Derivatives of such active polypeptides can include, for example, chemically or enzymatically modified polypeptides; fusion proteins; or polypeptides bound to a suitable carrier substance such as a polymer, etc. According to the present invention a method

for isolating, purifying and characterizing the P3 proteins and a method of using one or more of the P3 proteins and/or osteogenically active polypeptides and/or immunologically related (that is, immunologically related to one or more of the P3 proteins) osteogenically active entities as pharmaceutical agents for the stimulation of bone growth in mammals are disclosed. Pharmaceutically acceptable compositions comprised of one or more of the P3 proteins and/or osteogenically active polypeptides and/or immunologically related osteogenically active entities in combination with a pharmaceutically acceptable carrier are also disclosed herein. Such compositions can optionally contain other bioactive materials or other ingredients which aid in the administration of the composition or add to the effectiveness of the composition.

As used herein, the term "immunologically related" is meant to include any polypeptide which shows binding and/or recognition to antigen-binding sites in antibodies raised or manufactured against the protein. The term "osteogenesis" means formation of new bone or induction of growth of pre-existing bones at specific sites in response to local administration (for example, implantation) of an osteogenically active preparation in a pharmaceutically acceptable manner. The term "osteogenic amount" refers to an amount of the osteogenic P3 protein and/or osteogenically active polypeptide and/or immunologically related osteogenically active entity sufficient to provide the desired effect. The term "osteogenically active" or "osteogenic" means that the preparation has the capability to promote or induce osteogenesis.

Brief description of the drawings

Figure 1 represents the elution profile obtained by Sepharose® CL-6B column chromatography of the proteins obtained in an eight hour extraction of demineralized calf bone powder with 4 M GuHCl—0.01 M Tris · HCl buffer (pH 7.0).

Figure 2 represents the elution profile obtained by Sephacryl® S-200 column chromatography, in 4 M GuHCl—0.01 M Tris · HCl buffer (pH 7.0), of the proteins contained in the active fraction obtained from Sepharose CL-6B column chromatography.

Figure 3 represents the elution profile of proteins present in the active pool from Sephacryl® S-200 column chromatography on a reverse phase Protesil® 300 octyl column using an acetonitrile gradient for the elution of proteins.

Figure 4 represents the results of electrophoretic analysis of purified bone matrix proteins on discontinuous sodium dodecyl sulfate-polyacrylamide gels in the presence of a reducing agent.

Figure 5A represents the elution profile obtained by high performance liquid chromatography, on a reverse phase C8 column, of fragments of porcine P3 protein; the fragments were generated by the enzymatic digestion of porcine P3 protein using *Staphylococcus aureus* V8 protease.

Figure 5B represents the elution profile obtained by high performance liquid chromatography, on a reverse phase C8 column, of fragments of bovine P3 protein; the fragments were generated by the enzymatic digestion of bovine P3 protein using *Staphylococcus aureus* V8 protease.

Figure 6A represents the elution profile obtained by high performance liquid chromatography, on a reverse phase C18 column, of fragments of porcine P3 protein; the fragments were generated by the enzymatic digestion of reduced, carboxymethylated porcine P3 protein using *Staphylococcus aureus* V8 protease.

Figure 6B represents the elution profile obtained by high performance liquid chromatography, on a reverse phase C18 column, of fragments of human P3 protein; the fragments were generated by the enzymatic digestion of reduced, carboxymethylated human P3 protein using *Staphylococcus aureus* V8 protease.

Figure 7 represents the results of competitive radioimmunoassays measuring the ability of radiolabelled test antigen to bind to specific antibody molecules in the presence of competing unlabelled antigen preparations.

Detailed description of the invention

Using the procedures described herein, the primary osteogenic factor and several other proteins can each be purified to an essentially homogeneous state starting from the crude extract of the demineralized bone powder of a particular mammalian species. For example, from the bone of a particular mammalian species, a substantially pure preparation of the primary osteogenic factor can be obtained in addition to substantially pure preparations of several other proteins which do not promote bone formation in the absence of the primary osteogenic factor.

As judged by the migration of these substantially pure proteins in polyacrylamide gels under dissociating, reducing conditions, using the procedure essentially as described by Laemmli, U.K., Nature, Vol. 227, pp. 680—685 (1970), different protein species have been assigned names such as P1, P2, P3, P4 and the like in the order of decreasing apparent molecular weight (see Table 1). Equivalent proteins have been obtained from bones of different mammals. For example, equivalent proteins corresponding to the primary osteogenic factor with an assigned name of P3 herein, have been isolated from bones of several mammals. These proteins are representative members of the family of immunologically related P3 proteins; the P3 protein purified to an essentially homogeneous state obtained from calf bone according to the procedures essentially as described herein, has an apparent molecular weight of 22,000 to 24,000 daltons, and has an amino terminal sequence and an amino acid composition as described later herein.

Similarly, the P3 protein isolated from human bone and purified to an essentially homogeneous state according to the procedure essentially as described herein is immunologically related to the calf P3 protein and porcine P3 protein, has an apparent molecular weight of 22,000 to 24,000 daltons and an amino acid composition as described later herein. Likewise, the P3 protein isolated from porcine bone and purified to an essentially homogeneous state according to the procedures essentially as described herein is immunologically related to the calf P3 protein and human P3 protein, has an apparent molecular weight of 22,000 to 24,000 daltons and an amino acid composition as described later herein. Each of the P3 proteins, irrespective of bone tissue source, exhibits osteogenic activity.

In addition, two protein preparations, designated herein as P2 and P4, and unrelated to the P3 protein, have also been isolated from bone of each of several different mammalian species. A family of P2 proteins, each member isolated from a particular mammalian bone source, has been characterized. A typical P2 protein isolated from calf bone has an apparent molecular weight of 30,000 to 33,000 daltons, is incapable of inducing osteogenesis in the absence of a representative P3 protein, and has an amino terminal sequence as described later herein. Immunologically related members of this P2 protein family have been isolated according to the procedure essentially as described herein from human bone and porcine bone.

In a similar manner, a family of P4 proteins has been isolated according to the procedures described herein. In the stage of purification accomplished for the P4 protein from calf bone, the P4 preparation consists of two major components which are incapable of inducing osteogenesis in the absence of P3 protein, both having an apparent molecular weight of about 16,000 to 18,000 daltons and are characterized by amino terminal amino acid sequences as described later herein. Immunologically related members of this P4 protein family, also incapable of inducing osteogenesis in the absence of P3 protein, have been isolated from human bone and porcine bone according to the procedure essentially as described herein.

The invention also concerns molecular entities detected using antibodies prepared against any of the P3 proteins described herein; other proteins with molecular weights which are substantially different than the molecular weights of the P3 proteins have been detected in other mammalian tissues (that is, in tissues other than bone) by the ability of these other proteins to be recognized by the antibodies to P3 proteins. In particular, proteins having an apparent molecular weight of about 40,000 to about 45,000 daltons have been identified by this technique in tissues such as rat, human or bovine brains; calf or rat dental pulp; bovine, human or porcine cartilage of the trachea or joints; and cultured cells derived from rat bone tumors. As is known in the art, immunologically related proteins of apparent molecular weights substantially higher than a biologically active entity frequently represent biosynthetic precursors or covalently modified forms (such as glycosylated and/or acylated forms, etc.) of the active entity. Such precursors may or may not show biological activity but can often be processed by standard chemical or enzymatic means to the active entity. The 40,000 to 45,000 dalton proteins obtainable and identified by means described herein may comprise a distinct family of immunologically related proteins which may be osteogenetically active or activable.

Other proteins having apparent molecular weights of about 14,000 to about 16,000 daltons have been detected in several mammalian tissues by their ability to bind to antibodies generated against a P3 protein. These proteins might be derived from the P3 proteins or their precursors by specific or nonspecific cleavage by proteases. Some of these proteins may have osteogenic activity.

The specific antibodies generated against a P3 protein have also detected the presence of proteins having molecular weights of from about 22,000 to 26,000 daltons in mammalian tissues other than bone and in cultured cells derived from non-bone tissues; these proteins may be osteogenically active.

As used herein, the term "essentially homogeneous" is meant to describe a protein which is homogeneous by one or more purity or homogeneity characteristics normally used by those of skill in the art of protein chemistry. For example, an essentially homogeneous protein will show constant and reproducible characteristics within standard experimental deviations for parameters such as the following: amino acid analysis, amino- or carboxyl-terminal sequence, band pattern on conventional polyacrylamide gel electrophoresis (PAGE) or other chromatographic techniques, molecular weight, isoelectric point, immunological properties and other such parameters. The terms, however, are not meant to exclude artificial or synthetic mixtures of the protein with other proteins. Thus the present invention includes mixtures of two or more essentially homogeneous proteins, for example, mixtures of P3 and P4; of P3 and P2; of P2, P3 and P4; of P3 with neutral matrix protein(s); of P3 with other yet to be discovered osteogenic proteins; of P3's from two or more mammalian sources, and the like. The terms are also not meant to exclude the presence of minor impurities which do not interfere with the biological activity of the protein, and which may be present, for example, due to incomplete purification.

The application of the osteogenic substances of the present invention can be conveniently accomplished by administering, such as by implanting, a lyophilized preparation or suspension of one or more of the osteogenic P3 proteins and/or one or more osteogenically active polypeptides and/or one or more immunologically related osteogenically active entities in sufficient quantity to promote osteogenesis at the desired site. Alternatively, pharmaceutically acceptable compositions can be used which are comprised of one or more of the osteogenic P3 proteins and/or one or more of the osteogenically active polypeptides and/or one or more of the immunologically related osteogenically active entities described herein and a pharmaceutically acceptable matrix such as collagenous proteins or matrix material derived

from powdered bone extracted with strong denaturing agents, or other pharmaceutically acceptable carriers.

The procedure essentially as outlined in the following example can be used to obtain substantially pure preparations of the respective P3 proteins; the particular P3 protein which is obtained simply depends upon which mammalian species the bone is obtained from. The example is included to further illustrate the invention but is not to be construed as a limitation thereon.

Example

Isolation of the osteogenic factor(s)

Bone processing

In a typical preparation, long bones (ends of long bones) from a mammal (for example, ankles from calves, femur heads or vertebral column from human bones, the total tibia and fibula from rats) are processed and demineralized using well known conventional procedures such as those described in Urist, M. R., U.S. Patent No. 4,294,753 (1981). These and all other references cited herein are incorporated herein by reference.

A convenient method of processing and demineralizing bone is as follows:

The periosteal layer surrounding the bone (preferably the bone is obtained from a young mammal and kept refrigerated until processing) is removed by mechanical means and then the marrow from the central cavity of the bone is removed by washing with cold water. The bone is pulverized into small particles [generally 1 to 2 millimeters (mm) in diameter] by conventional means, for example, using a Wiley mill. The particles are then washed extensively with a buffered saline solution such as a 0.15 M NaCl-0.1 M Tris · HCl buffer (pH 7.0) to remove most of the lipids and remaining blood. The particles are further reduced in size by shearing, for example, using a polytron homogenizer (Brinkman Instruments) so that particles of approximately 500 μm in diameter or less are obtained. The homogenized particles are washed with buffered saline such as that noted above and water, then with ethanol and finally with ether. The washed homogenized particles are then vacuum or air dried; this "bone powder" can be stored at −80°C for prolonged periods of time.

For efficient demineralization and protein extraction the bone powder is sieved to obtain particles having a size range of about 75 to 500 μm in diameter. Demineralization (that is, the removal of calcium phosphate from the bone matrix) is achieved by repeated washes with a hydrochloric acid (HCl) solution, for example, by stirring bone powder for one hour with about 10 to 15 milliliters (ml) of 0.5 normal (N) HCl per gram (g) dry weight of bone powder, decanting the liquid and then repeating this process three or four times. The demineralized bone powder is then washed extensively with deionized distilled water until the pH approaches neutrality. The water is removed from the demineralized bone powder by washing with ethanol, then ether, and then drying. The demineralized bone powder can be stored at low temperatures (for example, −20° to −80°C). Demineralization of the bone powder can also be accomplished using other well known procedures, for example, using a chelator such as ethylenediaminetetraacetic acid.

To determine if the treated bone powder is sufficiently demineralized after HCl treatment to be ready for the extraction of the bone-matrix proteins, the water-rinsed powder is tested for mineral content [(that is, calcium content), for example, by the silver nitrate staining method of von Kossa, see J. von Kossa, Ziegler's Beitr. *29*, 163 (1901)]. When the von Kossa stain is negative the treated bone powder is sufficiently demineralized to be ready for the extraction of proteins. Such demineralized bone powder when implanted in test animals induces the formation of new bone at the site of implantation, that is, contains putative, but unidentified, osteogenic factors.

Extraction and separation of proteins from demineralized bone powder

Demineralized bone powder, prepared as described above, is extracted by constant stirring with an aqueous solution of about 2 to 8 molar (M) guanidinium-hydrochloride (GuHCl) in a buffer such as Trizma-hydrochloride (Tris · HCl) at or near pH 7.0 for a time sufficient to extract the desired proteins. Preferably, the extraction is performed by stirring the demineralized bone powder with 4 M GuHCl-0.01 M Tris · HCl buffer (pH 7.0) in the presence of a proteolytic enzyme inhibitor such as phenylmethylsulfonylfluoride for 8 to 12 hours (hrs) between about 4° to 20°C. The proteins from demineralized bone powder can be extracted by contacting the demineralized bone powder with an appropriate GuHCl-Tris · HCl buffer for a time sufficient to obtain substantial quantities of the desired proteins. In a typical extraction of 100 grams of demineralized calf bone powder, approximately 1500 milligrams (mg) of total proteins are extracted in a three day extraction period with 4 M GuHCl-0.01 M Tris · HCl buffer (pH 7.0). In the process of the present invention, it has been found that more than 80 percent (%) of the total proteins obtained in a three day extraction can be extracted in the first 8 to 12 hrs with a 4 M GuHCl-0.01 M Tris · HCl buffer (pH 7.0). During the first 8 to 12 hrs of extraction typically more than 95% of the total low molecular weight protein population that can be obtained in a three day extraction is recovered. Most osteogenic activity is associated with these low molecular weight proteins. About 15 ml of the 4 M GuHCl-0.01 M Tris · HCl buffer (pH 7.0) solution is used per gram dry weight of demineralized bone powder. After the extraction period is complete, the extract is filtered, for example, over Whatman paper, and the filtrate concentrated by conventional procedures; in typical experiments, an Amicon ultrafiltration apparatus (Amicon Corporation, Lexington, Massachusetts) with a membrane filter

or a hollow fiber filtration cartridge with molecular cut-off size of approximately 5,000 daltons is used for the concentration step (that is, the membrane or hollow fiber cartridge retains molecules having a molecular weight greater than approximately 5,000 daltons, for example, an appropriate Diaflo® ultrafiltration membrane such as YM-5 or a hollow fiber cartridge such as H1P5-20 from Amicon Corporation).

The various buffers, for example, the 4 M GuHCl-0.01 M Tris · HCl buffer, and solutions, for example, the 0.5 N HCl solution, described herein are aqueous buffers or solutions in which the indicated materials are present in water at the indicated concentration. The protein components of the concentrated protein solution were fractionated using various conventional chromatographic techniques including high performance liquid chromatography (HPLC) as follows:

The initial protein fractionation was conveniently accomplished by chromatography on a Sepharose CL-6B (Pharmacia Chemicals, New Jersey) column. In a typical experiment, the proteins extracted as described herein are concentrated by ultrafiltration to a concentration of about 25 to 40 mg/ml. The concentration of proteins in various extract preparations and column fractions were usually estimated by conventional means such as spectrophotometric measurement of the absorbance of the solutions at 280 nanometers (nm). An appropriate amount of the protein concentrate (an amount providing approximately 500 mg of protein) was applied to a 5 centimeter (cm)×90 cm Sepharose CL-6B column equilibrated with 4 M GuHCl-0.01 M Tris · HCl buffer (pH 7.0). The column is eluted with the 4 M GuHCl-0.01 M Tris · HCl buffer (pH 7.0) at a hydrostatic pressure head of between about 50 to 100 cm and individual fractions of 15 to 20 ml volume collected. A typical elution profile under the above conditions was obtained by measuring the absorbance of individual fractions at 280 nm and is shown in Figure 1.

The bone inducing activity of various fractions eluted from the Sepharose CL-6B column was measured, using the bone induction assay system described herein, and indicated that the pool of fractions identified as "C" in Figure 1 contained the factors responsible for the osteogenic activity. Pool C, which consisted of pooled fractions V, VI and VII, was concentrated using conventional procedures. In a standard extraction, pool C obtained from the elution of the total proteins on the Sepharose CL-6B column represents about 40% of the total proteins obtained in an 8 to 12 hr extraction of demineralized calf bone powder with 4 M GuHCl-0.01 M Tris · HCl buffer (pH 7.0). Further fractionation was then achieved by chromatography on a Sephacryl S-200 (Pharmacia Chemicals, New Jersey) column. In a typical experiment, 75 to 100 mg of proteins from pool C are applied at a concentration of approximately 25 mg/ml to a 2.2 cm×115 cm Sephacryl S-200 column and the column eluted with 4 M GuHCl-0.01 M Tris · HCl buffer (pH 7.0) under a hydrostatic pressure head of between about 50 to 75 cm and individual fractions of approximately 4 ml in volume collected. A typical elution profile which was obtained under the above conditions is shown in Figure 2.

Fractions from the Sephacryl S-200 column were pooled (see Figure 2) and the resulting pooled materials arbitrarily identified as alpha (α), beta (β), gamma I (γI), gamma II (γII) and delta (δ).

Analysis of the proteins, using conventional discontinuous polyacrylamide gel electrophoresis in the presence of sodium dodecyl sulfate [Laemmli, U.K., Nature, Vol. 227, pp. 680—685 (1970)], contained in the respective alpha through delta pools allowed identification of several proteins. It was found that the alpha pool contained minor protein components of molecular weight higher than 50,000 daltons; the beta pool contained a major species at 38,000 to 40,000 daltons, some minor higher molecular weight contaminants, and small quantities of lower molecular weight protein species migrating between 14,000 and 30,000 daltons; the gamma I and gamma II pools contained four major size class species migrating at 31,000 to 35,000 daltons, at 22,000 to 25,000 daltons, at 16,000 to 18,000 daltons, and at 12,000 to 14,000 daltons; the delta pool contained mostly proteins in the 12,000 to 14,000 dalton range.

Measurement of activity in the bone induction assay essentially as described herein indicated that the gamma I and gamma II pools contained factors inducing bone formation.

To simplify the discussion concerning the final purification of the proteins a list of the protein species found in the beta, gamma and delta pools is presented in Table 1. As indicated previously, each of the respective major protein species was assigned an identifying name (P1, P2 and the like) as indicated in Table 1.

TABLE 1

| Major species | | Minor species | |
|---|---|---|---|
| Assigned name | Estimated molecular weight×$10^{-3}$ | Assigned name | Estimated molecular weight×$10^{-3}$ |
| P1 | 38—40 | | |
| P2 | 30—33 | | |
| | | PA | 28—30 |
| | | PB | 24 |
| P3 | 22—24 | | |
| | | PC | 19 |
| P4 | 16—18 | | |
| P5a | 13—14 | | |
| P5b | 14* | | |
| | | PD | 12 |

All primary molecular weight assignments of protein species are based on mobilities in discontinuous polyacrylamide gel electrophoresis with 13% acrylamide at pH 8.8 in the resolving gel in the presence of sodium dodecyl sulfate and a reducing agent. The minor protein species represented less than 10 to 15 percent of the total material in the respective samples analyzed on gels, *P5b migrates at about 10,000 daltons under non-reducing conditions which serves to distinguish P5a from P5b.

Final purification of the P3 protein

The final purification was accomplished by reverse phase HPLC of the partially purified protein preparations, obtained from Sephacryl S-200 column chromatography, using a Beckman Altex HPLC controlled by a Model 421 microprocessor unit. Two approaches have been used.

A characteristic feature of some of the proteins, especially the P3 protein family described herein is the lack of solubility in the absence of a strong dissociating agent such as GuHCl. In addition, when multiple protein species were simultaneously present in a pool, the removal of GuHCl resulted in a coprecipitation of other proteins along with P3. A method was, therefore, developed where narrow pools consisting of only one or two major proteins were obtained from the Sephacryl S-200 column and used as the starting material for further purification by HPLC. In addition, in order to maximize the retention of proteins in solution, pools such as the ones described above were dialyzed directly against an aqueous solvent containing 0.1% trifluoroacetic acid (TFA) supplemented with acetonitrile (ACN) at concentrations of between 10% to 15% by volume. A conventional dialysis membrane tubing with molecular weight cut-off size of 3,500 daltons or lower is conveniently used in this procedure. Proteins soluble in the TFA:ACN solvent could then be conveniently obtained by removal of the insoluble material from each dialyzed pool by centrifugation. The soluble proteins at this point could be chromatographed on a reverse phase HPLC column such as the Protesil 300 octyl column described herein. In a typical experiment, the TFA:ACN soluble proteins obtained from the peak fractions in this manner were applied to a 0.46 cm×25.0 cm Protesil 300 octyl column (Whatman) of 10 µm particle size equilibrated with 0.1% TFA:10% ACN. Proteins bound to the column under these conditions were eluted at a flow rate of 60 ml/hr using a linear 10% to 80% ACN gradient developed over 45 minutes. In a typical experiment, as indicated in Figure 3A, P2 and P1 proteins were sequentially recovered with increasing ACN concentrations (depicted by the dashed line) from the gamma I peak. Similarly, P1 protein can be obtained from the beta peak while P5a and P5b are obtained from the delta peak. The P3 protein elutes between the gamma I and gamma II regions of the Sephacryl S-200 column. The P3 protein is found in both the soluble and the insoluble materials obtained by dialysis of appropriate fractions against TFA:ACN. The lack of solubility of the P3 protein thus yields essentially homogeneous P3 protein in the insoluble material. The P3 protein retained in solution in the TFA:ACN solvent can be further purified by reverse phase HPLC essentially as described above.

The second procedure to purify proteins to an essentially homogeneous state was designed to take advantage of the high degree of insolubility of certain proteins in the 35,000 to 14,000 dalton molecular weight range, especially when they are present together at high concentrations (for example, approximately 10 mg/ml). In this procedure proteins eluting in the gamma I and gamma II pools from the Sephacryl S-200 column chromatography (that is, the pools where the bone inducing activity is found) were concentrated to approximately 10 mg/ml. The material was rapidly dialyzed [for example, six changes each of 4 liters every 2 to 3 hrs, (using dialysis tubing with a molecular cut-off size of 2,000 daltons)] against deionized distilled water at 15° to 23°C. Precipitated proteins were collected by centrifugation and washed several times with deionized distilled water keeping the concentration of protein at higher than 10 mg/ml of washing water. The principal constituents of this precipitated material were found to be P2, P3, P4 and P5a; small amounts of P1 protein was found in variable quantities in some cases. The final pellet was dissolved

in 0.1% TFA with 15% ACN and the solubilized material was applied to a Protesil 300 octyl column. Increasing ACN concentration eluted the P2, P3, P4 and P5a proteins as shown in Figure 3B, a typical elution profile.

Each of the major protein species described in Table 1 was further purified by rechromatographing on the Protesil 300 octyl column. Pools of fractions obtained as indicated in Figure 3 were concentrated by lyophilization and redissolved in 0.1% TFA and about 10 to 20% ACN depending upon the particular lyophilized material and reapplied to the Protesil 300 octyl column. The proteins were eluted from the column using a linear 10% to 80% ACN gradient at a flow rate of 60 ml/hr under conditions as previously described herein except that the proteins were eluted over a longer period thus resulting in numerous individual fractions. The purity of each of the protein fractions was determined using conventional discontinuous PAGE. Those fractions which showed only one major species were used for further chemical and biological characterizations. Typically these fractions were lyophilized and stored as lyophilized powders.

Figure 4 depicts the results of a typical discontinuous gel electrophoretic analysis on sodium dodecyl sulfate-polyacrylamide gels. The analysis was performed on a discontinuous polyacrylamide gel system in the presence of sodium dodecyl sulfate and a reducing agent where the resolving gel was 13% in acrylamide and 0.35% in bis-acrylamide crosslinker at a pH of 8.8. The gel was run at 50 volts for 30 minutes followed by 7 hrs at 100 volts. Protein bands were visualized by staining with coomassie brilliant blue R. Columns 1 and 8 depict gels with the following standard molecular weight markers: 95,000 (phosphorylase A), 68,000 (bovine serum albumin), 43,000 (ovalbumin), 31,000 (carbonic anhydrase), 21,000 (soybean trypsin inhibitor), and 14,000 (ribonuclease); Columns 2, 3, 4, 5 and 6 show, respectively, the P1, P2, P3, P4 and P5 proteins (CP1 thru CP5) from demineralized calf bone powder; and Column 7 the P3 protein (HP3) from demineralized human bone powder. Portions shaded with oblique lines are bands of low concentration.

The amino terminal sequences of the P2, P3 and P4 proteins obtained from calf bone were investigated using the following procedure:

Amino terminal sequences were determined on an Applied Biosystems Model 470A Gas Phase Protein Sequencer. Reconstituted samples were applied to the loading disc in a volume of 30 microliters (μl) in a solvent appropriate to dissolve the protein (typically trifluoroacetic acid). Polybrene was added to the disc prior to the addition of the sample and the reaction vessel precycled for 12 cycles to condition the Polybrene/disc carriers. The Edman degradation and resultant conversions were performed automatically by the instrument. The amino acid derivatives were identified on a Hewlett-Packard High Performance Liquid Chromatograph Model 1084B using an acetate/acetonitrile:methanol gradient as described previously, see Thomas, K. A., et al., J. Biol. Chem., Vol. 256, pp. 1947—1955 (1981). A Beckman C18 microsphere reverse phase column was employed for the identification. The gas sequencer and HPLC program employed allowed identification of all 20 amino acid derivatives in each sample.

The partial amino terminal sequence of the calf P3 protein (calf P3 protein is also referred to herein as "bovine P3 protein") was determined to be $H_2N$-Phe-Pro-Val-Tyr-Asp-Tyr-Ser-Pro-Ala-Arg-Leu-Lys-Glu-Ala.

The partial amino terminal sequence of the calf P2 protein was determined to be $H_2N$-Trp-?-Pro-Tyr-?-Trp.

The partial amino terminal sequence of the calf P4 protein complex indicated that two major components were present with the following sequences:

$H_2N$-Ala-Glu-Pro-?-?-Tyr;
$H_2N$-Pro-Glu-Pro-?-?-Tyr.

Conventionally accepted abbreviations are used herein to represent the respective amino acids as follows: Ala, alanine; Arg, arginine; Asn, asparagine; Asp, aspartic acid; Cys(½), cysteine; Gly, glycine; Gln, glutamine; Glu, glutamic acid; His, histidine; Ile, isoleucine; Leu, leucine; Lys, lysine; Met, methionine; Phe, phenylalanine; Pro, proline; Ser, serine; Thr, threonine; Tyr, tyrosine; Trp, tryptophan; Val, valine. As used in the amino acid sequences the "?" indicates an amino acid which remains unidentified.

Following the extraction and purification procedure essentially as described herein, a protein was obtained from demineralized human bone powder having a molecular weight of approximately 23,000 daltons and designated as human P3 protein. Human P3 protein purified to an essentially homogeneous state induced the formation of bone when implanted. The human P3 protein obtained from human bone is related to the calf P3 protein. Following the extraction and purification procedure essentially as described herein, a protein was obtained from demineralized porcine bone powder having a molecular weight of approximately 23,000 daltons and designated as porcine P3 protein. Porcine P3 protein obtained from porcine bone is related to the calf P3 and human P3 proteins. The respective calf, human and porcine P3 proteins show the following similarities:

(1) all are extracted and purified following the procedures described herein, all are only sparingly soluble in water in the absence of a strong dissociating agent;

(2) each has the demonstrated ability to induce bone formation as judged by alkaline phosphatase activity measurements and by histological examination of explant tissues which developed as the result of the implantation of the protein;

(3) each has approximately the same apparent molecular weight in the discontinuous sodium dodecyl sulfate-polyacrylamide gel electrophoresis system;

(4) the proteins are immunologically related since antibodies prepared against the calf bone-derived P3 protein bind to human and porcine bone-derived P3 proteins; similarly, antibodies prepared against the porcine P3 protein or the human P3 protein bind all three P3 proteins;

(5) amino acid analyses show significant similarities as described in Table 2; and

(6) the amino acid sequences of peptides generated from the respective P3 proteins indicate regions of extensive homology, see Table 4.

Amino acid compositions for the calf, the porcine and the human P3 proteins were determined from acid hydrolysates prepared with redistilled 6N hydrochloric acid (110°C, 24 hrs). The tubes were evacuated prior to sealing to eliminate oxygen. Following removal of the 6N hydrochloric acid by evaporation and reconstitution in citrate buffer, the hydrolysates were analyzed, on a Beckman Model 6300 automatic amino acid analyzer. Standard operating procedures were used as described by Benson, J. R., et al., "Amino Acid Analysis of Peptides" in *Peptides: Analysis, Synthesis, Biology* (E. Gross and J. Meienhofer, eds.) Academic Press, New York, Vol. 4, pp. 217—260 (1981). The quantitative data produced was converted to residues/mole of protein based upon the molecular weights estimated by conventional PAGE.

Table 2 presents amino acid composition data obtained from the human P3, the porcine P3 and the calf P3 proteins. The data represents the average values obtained from six runs per protein (triplicate analyses on each of two independently purified preparations) on a Beckman Model 6300 amino acid analyzer. It should be noted that determinations of amino acid compositions are subject to errors introduced by (i) the extent of protein hydrolysis where the yields of certain amino acids vary because of variations in stability and/or hydrolyzability of different amino acid residues, (ii) minor impurities whose exact amounts vary from one preparation to another, (iii) computerized data analysis derived from the peak-areas corresponding to the positions of different amino acids, and (iv) errors inherent in the estimation of the exact molecular weights of proteins by conventional techniques such as SDS-PAGE or column chromatography.

TABLE 2
Amino acid composition of the P3 proteins obtained from the bone of different mammalian species

| | Number of residues in Human P3 protein | Porcine P3 protein | Calf P3 protein |
|---|---|---|---|
| Aspartic Acid & Asparagine | 21 | 24 | 23 |
| Threonine | 9 | 9 | 8 |
| Serine | 11 | 14 | 12 |
| Glutamic Acid & Glutamine | 30 | 30 | 28 |
| Proline | 10 | 13 | 8 |
| Glycine | 18 | 18 | 20 |
| Alanine | 9 | 7 | 8 |
| Valine | 9 | 10 | 8 |
| Methionine | 5 | 9 | 4 |
| Isoleucine | 6 | 5 | 5 |
| Leucine | 8 | 6 | 8 |
| Tyrosine | 20 | 24 | 16 |
| Phenylalanine | 7 | 8 | 8 |
| Histidine | 2 | 3 | 4 |
| Lysine | 5 | 5 | 5 |
| Arginine | 10 | 9 | 19 |
| Tryptophan | (10) | (7) | (4) |
| Cysteine | (6) | (7) | (6) |

The numbers represent approximate number of residues (to the nearest whole number) of the indicated amino acids per mole of the respective proteins whose molecular weights were estimated by independent methods conventionally used, such as polyacrylamide gel electrophoresis. The numbers for the P3 proteins of this invention are within experimental errors inherent in the art of amino acid composition analysis. Values in parentheses were determined based upon absorbance at specific wavelengths.

The amino acid composition data indicates the estimated number of residues of each amino acid per molecule of the respective P3 proteins. As appreciated by one skilled in the art, the accuracy of amino acid composition data is dependent upon the number of test runs and the extent of protein purification. The amino acid composition data indicates that the compositions of human, porcine and calf P3 proteins are similar to each other, for example, each has high contents of aspartic acid (+asparagine) and glutamic acid (+glutamine) residues, as well as substantial numbers of tyrosine residues.

Table 3 contains the amino acid compositions of several proteins previously described in the art, namely, a 17,000 to 18,000 dalton bone morphogenetic protein from calf bone described by Urist, M. R., et al., Science, Vol. 220, pp. 680—686 (1983), see "Calf[1]" in Table 3; a 17,000 to 18,000 dalton bone morphogenetic protein from human bone, Urist, M. R., et al., Proceedings of The Society of Experimental Biology and Medicine, Vol. 173, pp. 194—199 (1983), see "Human[2]" in Table 3; a 23,000 dalton bone morphogenetic protein from rabbit dentin, Conover, M. A., and Urist, M. R., The Chemistry and Biology of Mineralized Connective Tissues, Elsevier North Holland, Inc., pp. 597—606 (1981), see "Rabbit[3]" in Table 3; a bovine bone-derived protein said to have osteogenic activity having a molecular weight of 18,500 daltons, Urist, M. R. et al., Proc. Natl. Acad. Sci. USA, Vol. 81, pp. 371—375 (January, 1984), see "Bovine Bone 18.5K

Protein[4]" in Table 3; and a calf bone-derived protein which does not induce osteogenesis and has a molecular weight of 24,000 daltons, Urist, M. R., et al., Science, Vol. 220, pp. 680—686 (1983), see "Calf Bone 24K Protein[5]" in Table 3.

TABLE 3*
A comparison of the amino acid compositions of several previously reported bone derived proteins

| | Previously reported proteins | | | | |
|---|---|---|---|---|---|
| Amino acid | Calf[1] | Human[2] | Rabbit[3] | Bovine bone 18.5K protein[4] | Calf bone 24K protein[5] |
| Asp (+Asn) | 17 | 44 | 34 | 18 | 30 |
| Thr | 7 | 5 | 8 | 7 | 13 |
| Ser | 19 | 14 | 25 | 17 | 20 |
| Glu (+Gln) | 18 | 18 | 28 | 25 | 18 |
| Pro | 12 | 7 | 9 | 10 | 13 |
| Gly | 15 | 43 | 17 | 16 | 20 |
| Ala | 11 | 12 | 9 | 11 | 16 |
| Val | 9 | 8 | 4 | 9 | 10 |
| Met | 3 | 2 | 1 | 2 | 4 |
| Ile | 8 | 6 | 4 | 4 | 8 |
| Leu | 12 | 12 | 7 | 11 | 14 |
| Tyr | 7 | 5 | 5 | 6 | 12 |
| Phe | 8 | 5 | 5 | 6 | 11 |
| His | 3 | 3 | 3 | 3 | 8 |
| Lys | 7 | 7 | 10 | 6 | 6 |
| Arg | 8 | 12 | 8 | 12 | 6 |
| Cys (1/2) | 3 | 4 | 1 | 5 | 0 |
| Trp | N.D. | N.D. | N.D. | N.D. | N.D. |

* The numbers in the table represent approximate number of residues (to the nearest whole number) of the indicated amino acids per mole of the respective proteins whose molecular weights were estimated by independent methods conventionally used, such as polyacrylamide gel electrophoresis. N.D. means no determination was made of the indicated amino acid.

[1] A 17,000 to 18,000 dalton bone morphogenetic protein from calf bone.
[2] A 17,000 to 18,000 dalton bone morphogenetic protein from human bone.
[3] A 23,000 dalton bone morphogenetic protein from rabbit dentin.
[4] A 18,500 dalton bone morphogenetic protein from bovine bone.
[5] A 24,000 dalton protein from calf bone which does not induce osteogenesis.

The amino acid composition data presented herein demonstrates that the P3 family of osteogenic factors are distinct from all other biologically active proteins that are claimed to be involved in osteogenesis and disclosed in the prior art.

P3 Proteins obtained from the bone of different mammalian species exhibit substantial chemical similarity
Proteolytic cleavage patterns

Proteases, enzymes that digest protein molecules into smaller polypeptide fragments, cleave peptide bonds with a certain degree of specificity. For example, the enzyme trypsin cleaves peptide bonds after arginine and lysine residues while the V8 protease from *Staphylococcus aureus* cleaves peptide bonds after aspartic acid and glutamic acid residues. Thus, if two proteins are similar in their amino acid sequences, the fragments generated from one protein will have homologous counterparts in the other protein when each protein is digested with the same protease. The proteolytic cleavage patterns of the P3 proteins were studied using V8 protease from *Staphylococcus aureus*.

In one experiment the porcine P3 and the calf P3 proteins were treated in parallel with *Staphylococcus aureus* V8 protease, and the digestion product subjected to a strong reducing treatment in order to release fragments that might be held together by disulfide bonds. Following the reducing treatment, the peptide fragments thus generated were subjected to reverse phase HPLC on a Synchropak C8 column. The peptide fragments were eluted using a linear gradient, from 20% to 70% by volume of acetonitrile in 0.1% trifluoroacetic acid in water. The elution of the peptides was monitored by measuring the absorbance of the effluent at 229 nm. The reverse phase HPLC elution profile of the V8 protease digested and reduced porcine P3 protein on the Synchropak C8 column is shown in Figure 5A; and the elution profile for the comparably treated bovine P3 protein is shown in Figure 5B.

In a second experiment, porcine P3 and human P3 proteins were each first treated with a reducing agent, and the free sulfhydryl groups were chemically derivatized by S-carboxymethylation using techniques well known in the art. The S-carboxymethylated protein preparations were each digested with *Staphylococcus aureus* V8 protease. The peptide fragments which resulted from the V8 protease digestion were subjected to reverse phase HPLC on a Whatman C18 column. The peptides were eluted using a linear acetonitrile gradient, from 20% to 70% by volume in 0.1% TFA in water. The elution of the peptides was monitored by measuring the absorbance of the effluent at 229 nm. The reverse phase HPLC elution profile of the S-carboxymethylated and V8 protease digested porcine P3 protein on the Whatman C18 column is shown in Figure 6A, and the elution profile for the comparably treated human P3 protein is shown in Figure 6B.

Additional amino acid sequence information

A convincing test of relatedness between two or more proteins is obtained by examining the amino acid sequences of the proteins. The amino acid sequences of various portions of the P3 proteins obtained from calf, pig and human bone have been compared. The amino acid sequence data was obtained employing conventional procedures, such as gas phase microsequencing, on the intact P3 proteins or peptide fragments obtained therefrom.

The intact P3 proteins from human and porcine bone did not yield any amino acid sequence information, most likely indicating that the amino terminal amino acid residue in each of these protein preparations was blocked; however, it was possible to obtain amino acid sequence information when the calf P3 protein was analyzed to determine its amino terminal amino acid sequence (see the amino terminal amino acid sequence for calf P3 protein previously provided herein). As is known in the art of protein chemistry, modifications which block the amino terminal amino acid residue may be physiological, i.e., take place during the biosynthesis of the proteins in the tissues or may sometimes occur during the process of purification. In order to obtain additional amino acid sequence information, various peptide fragments, obtained by *Staphylococcus aureus* V8 protease digestion of the respective P3 proteins as described previously herein, were isolated and sequenced. The amino acid sequence data concerning the isolated peptides is summarized in Table 4. The information presented in Table 4 shows that homologous peptide fragments from calf, human and porcine P3 proteins have virtually identical amino acid sequences which indicates that mammalian osteogenic factors (i.e., P3 proteins obtained from different mammalian bone sources) are related proteins.

TABLE 4*

Amino acid sequences from several homologous domains of P3 proteins isolated from different mammalian bone tissue sources

| | |
|---|---|
| Calf: | -Met-Asp-Met-Ile-Arg-Tyr-?-Tyr-Asp- |
| Porcine: | -Met-Asp-Met-Ile-Arg-Tyr-?-Tyr- |
| Calf: | -Tyr-Tyr-Met-Arg-Gly-Ala-Thr-Thr-Thr-Phe- |
| Porcine: | -Tyr-Tyr-Met-Arg-Gly-Ala-Thr-Thr-Thr-Phe-Ser-Ala-Val- |
| Human: | -Tyr-Tyr-Met- |
| Porcine: | -Ile-Asn-Arg-Ala-Gly-Met-Gln-Trp-Tyr-Gln-Thr-?-?-Asn-Asn-Gly-Leu-Val-Ala-Gly-Phe-Lys-Tyr- |
| Human: | -Ile-Asn-Arg-Ala-Gly-Met-Gln-?-Tyr-Gln- |
| Porcine: | -Gly-Tyr-Asp-Ala-Gln-Trp-Asn-Tyr-Ala-Ser-Met-Pro-His-Pro-Gln-?-Leu- |
| Human: | -Gly-?-Asp-Arg-Gln-Trp-Asn-Tyr-Ala-?-Met-Pro-?-Pro-Gln-?-Leu-Gly-?-Thr- |

* The identity of the respective amino acid residues are represented by conventionally accepted abbreviations;
"?" represents residues which are presently unidentified.

Demonstrations of immunological relatedness of P3 proteins

P3 proteins obtained from bone of different mammalian species have been used to immunize laboratory animals, such as rabbits, in order to generate monospecific antibodies capable of binding to the respective P3 proteins. In a typical experiment, approximately 100 micrograms of the particular P3 protein to which antibodies are to be raised is admixed with Freund's complete adjuvant and inoculated into the footpads and at subcutaneous sites in rabbits; ten to fourteen days later a comparable amount of protein admixed with incomplete Freund's adjuvant is inoculated subcutaneously; after an additional ten to fourteen days the animal is inoculated with an additional 50 to 100 μg of protein mixed in a ten percent solution of aluminum hydroxide. The animal is subsequently immunized at four week intervals with 50 to 100 μg of protein.

The specific antibody titers developed against the respective P3 proteins have been measured by (a) an enzyme linked immunosorbent assay (ELISA) and by (b) the ability of the antibody to immunoprecipitate radiolabelled P3 protein molecules. In a typical ELISA assay 10 to 20 nanograms of the test antigen (that is, the particular P3 protein being tested) is bound to a plastic surface by air-drying of a protein solution within the wells of microtiter dishes (Falcon Products or Bellco Products). Serial dilutions of test antisera are incubated within the wells, unbound antibodies are removed by washing and the bound antibodies are then incubated with an enzyme conjugated second antibody preparation directed against the immunoglobulins of the species in which the test antiserum was generated. The amount of enzyme bound in each well is then quantitated by an appropriate color assay. In such testing, sera with high titers of antibody against the test antigen can be diluted several thousand fold and will still show significant color development.

In the other assay, that is, the radioimmunoprecipitation assay (RIP), the test antigen is labelled with a radioisotope such as $^{125}I$. A fixed quantity of the radiolabelled antigen is then incubated with serial dilutions of the test antisera. The immunecomplexed antigen is precipitated either using a second antibody directed against the immunoglobulins of the species in which the test serum was generated, or using a fixed *Staphylococcus aureus* bacterial suspension, or using *Staphylococcus aureus* protein A immobilized onto beads. As in the previously noted ELISA procedure, serum with high titers of specific antibody can be diluted several thousand fold and still precipitate significant amounts of the radiolabelled antigen.

In either assay, the specific titers of antibodies in test sera samples is determined by subtracting the values (color or precipitated radioactivity) obtained with serum from nonimmunized animals or from animals immunized with a protein which is unrelated to the test protein. The specificity of the antisera as well as the immunological relatedness of different proteins can be estimated in either assay by examining the relative effects of serum dilutions on the extent of binding of the antigen.

The immunological relatedness between the P3 proteins obtained from calf, porcine and human bones was tested using the above described assays. The results of the testing are presented in Table 5.

TABLE 5

Titers of antibodies against the respective P3 proteins in serum from rabbits immunized with essentially homogeneous P3 proteins obtained from bones of different mammals

| Test antigen | ELISA Titer[1] of serum from rabbits immunized with | | | RIP Titer[2] of serum from rabbits immunized with | | |
|---|---|---|---|---|---|---|
| | Bovine P3 | Porcine P3 | Human P3 | Bovine P3 | Porcine P3 | Human P3 |
| Bovine P3 | 1:3000 | 1:200 | 1:200 | 1:1600 | 1:50 | 1:50 |
| Porcine P3 | N.D.[3] | N.D. | N.D. | 1:20 | 1:200 | 1:150 |
| Human P3 | 1:200 | 1:500 | 1:1000 | 1:20 | 1:100 | 1:200 |
| Bovine P1 | 1:10 | 1:10 | 1:10 | 1:10 | 1:10 | 1:10 |

[1] The ELISA titer in this table is represented as the dilution of antiserum that produces half the maximum color in ELISA assays using approximately 10 nanograms of antigen immobilized in each test well. Color developed using corresponding dilutions of a nonimmune serum sample were subtracted from the values obtained with the test antisera.

[2] The RIP titer in this table is represented as the dilution of antiserum which precipitates approximately 30% of the input antigen (50 nanograms) which had been radioiodinated to a specific activity of approximately $10^6$ disintegrations per minute per microgram protein.

[3] "N.D." means "not determined".

The results set forth in Table 5 clearly demonstrate that antibodies directed against one P3 protein can bind P3 proteins of other species which indicates that the respective mammalian P3 osteogenic factors are immunologically related to one another. The relatedness between the P3 proteins obtained from the bone of three different mammalian species (that is, the P3 protein obtained from calf bone, the P3 protein obtained from human bone, and the P3 protein obtained from porcine bone) was further tested by competition radioimmunoassays. Competition radioimmunoassays are based on the ability of unlabelled antigen molecules to compete with the binding of the radiolabelled test antigen to the specific antibody molecules raised against the test antigen. The extent of immunological relatedness between a protein and the test antigen is determined by the slope of displacement of radiolabelled test antigen bound to the antibody when increasing amounts of the unlabelled competing protein are added to the incubation mixture. In competition radioimmunoassays, the human P3 protein does not compete as effectively as the calf P3 protein in displacing radiolabelled calf P3 protein from binding to antibodies raised against the calf P3 protein (Figure 7A). In a parallel assay, both the human P3 and the porcine P3 proteins efficiently competed with the binding of radiolabelled porcine P3 protein to antibodies raised against porcine P3 protein; the calf P3 protein competed with a much lower efficiency (Figure 7B). In a third assay, porcine P3 protein was more effective than calf P3 protein in displacing radiolabelled human P3 protein from binding to antibodies raised against human P3 protein (Figure 7C). Since antibodies to porcine P3 protein immunoprecipitated the iodinated human P3 protein, the P3 proteins were compared for their ability to compete in a broad cross-species assay (Figure 7D). Calf P3 protein again showed the least efficient competition in this assay. The fact that the respective P3 proteins compete with each other in the binding of specific antibodies raised against a particular P3 protein demonstrates that the respective P3 proteins are immunologically related. This conclusion is further supported by the results of other competition radioimmunoassays in which unrelated antigens, including non-P3 proteins obtained from various mammalian bone sources, failed to compete with P3 proteins in the binding of specific antibodies raised against a particular P3 protein. In Figures 7A, 7B, 7C and 7D, the word "bovine" identifies curves concerning bovine P3 protein; "human" identifies curves concerning human P3 protein; and "porcine" identifies curves concerning porcine P3 protein.

The immunological relatedness between the P3 proteins was also tested by the ability of antibodies directed against a particular P3 protein to bind to "denatured" or "denatured and reduced" forms of P3 proteins of other species using the technique of immunoblotting. In the immunoblotting procedure, proteins are denatured by heating in sodium dodecyl sulfate in the presence or absence of a reducing agent. The denatured or denatured and reduced proteins are then subjected to denaturing polyacrylamide gel electrophoresis in the presence of sodium dodecyl sulfate (SDS-PAGE). The protein molecules are then transferred to a nitrocellulose membrane filter. This replica of the electrophoretically resolved protein bands is then incubated with antibodies directed against the respective proteins. The binding of antibody to a protein band is monitored by the ability of radiolabelled or enzyme conjugated protein A from *Staphylococcus aureus* to bind to the antibodies attached to the proteins on the nitrocellulose membrane. The location of the appropriate protein bands recognized by the antibody is then visualized by

autoradiography. This technique allows one to identify the molecular species which is recognized by a specific antibody preparation.

When calf P3 protein was mixed with a number of unrelated proteins, electrophoresed through sodium dodecyl sulfate-polyacrylamide gels and then analyzed by immunoblotting using rabbit antibodies raised against the calf P3 protein, only one band (which had migrated to a position indicating an apparent molecular weight of approximately 23,000 daltons as determined by its migration in the SDS-PAGE) was visualized by autoradiography.

The immunological relatedness between calf, porcine and human P3 proteins was also examined by the immunoblotting technique. Antibodies raised against either the calf P3 protein or the porcine P3 protein bound to P3 proteins obtained from calf, porcine and human bone. Furthermore, many fragments (generated by proteolytic digestions) of one P3 protein were recognized by antibodies raised against the P3 protein of another species. These results indicate that between the respective P3 proteins, there are conserved domains which can be detected immunologically; this is also supported by the amino acid sequence data.

The identification of other proteinaceous materials related to the P3 proteins

Protein factors that induce growth/regeneration of mammalian tissues are frequently biosynthesized at sites other than the target sites. In addition, the biosynthesis of these factors may proceed through a larger or modified form that can be converted to a biologically active species *in vivo* or *in vitro*. In mammals, the production of biologically active peptides often proceeds through a number of intermediates, for example, prepro-proteins or pro-proteins, having varying degrees of biological activity or no biological activity.

The identification of the P3 protein family of mammalian osteogenic factors permits one skilled in the art to detect, typically by immunological means, other related molecular entities in cultured cells and other tissues.

A convenient method for identifying other proteinaceous materials which may possess osteogenic activity employs the immunoblotting technology previously described herein; however, other technologies such as the use of radioimmunoassays may also be used. In a typical experiment a candidate tissue or cultured cell mass is extracted with strong dissociative agents, such as guanidine hydrochloride or guanidine isothiocyanate or sodium dodecyl sulfate, in the presence or absence of a reducing agent. The total extract or partially fractionated extracts are then subjected to SDS-PAGE and the electrophoretically separated protein species transferred onto nitrocellulose membrane filters. The presence of molecular species related to the P3 protein osteogenic factors are detected by their ability to bind antibodies directed against the P3 proteins.

In experiments using partially fractionated extracts of bovine brain, an immunologically related entity, with an apparent molecular weight of about 40,000 to 45,000 daltons, was detected using antibodies raised against calf P3 protein in certain fractions containing higher molecular weight entities; in fractions that contained the lower molecular weight proteins extracted from bovine brain a mixture of lower molecular weight entities immunologically related to calf P3 protein, having molecular weights in the range of about 14,000 to 25,000 daltons, were found.

Similar analyses performed on calf dental pulp tissues showed the presence of a 23,000 to 25,000 dalton molecular weight entity and lower levels of a 14,000 to 16,000 dalton entity and a 40,000 to 45,000 dalton entity each of which bound antibodies raised against the calf P3 protein. The presence of similar proteins has been detected in tracheal, nasal and articular cartilage tissues of calves, in tissues and cultured cells of a rat bone tumor, and in certain rat tissues induced to develop bone by the implantation of demineralized bone matrix. Osteogenically active or activable proteins or polypeptides detected by immunological techniques described herein and other osteogenically active or activable proteins or polypeptides present in other mammalian tissues detected by antibodies raised against a P3 protein osteogenic factor from a mammalian bone tissue source are considered to be within the scope of this invention.

The calf P3, the porcine P3 and the human P3 proteins in an essentially homogeneous state, each, when implanted in rats, as demonstrated in testing using the bioassay system described herein, induces the formation of bone at the implant site in approximately three weeks. It appears that the members of the P3 protein family purified from different mammals will have osteogenic activity in mammals in general. Thus the P3 proteins represent a family of immunologically related proteins considered to be primary osteogenic factors.

Bone induction assay system

To determine the osteogenic activity of test protein fractions or proteins a procedure such as the following can be used. Bone matrix powder (75 to 500 μm size) is demineralized as described herein and then extracted sequentially three times, each with 15 to 20 ml of 4 M GuHCl per gram of demineralized bone powder. The extracted matrix is extensively washed with water, followed by ethanol and ether and then the powder is dried. This powder, when implanted in a test animal, such as a rat, does not induce osteogenesis and is called inactive bone matrix (IBM). In order to measure the activity of a protein preparation, the IBM powder is mixed with an aqueous solution or suspension of the protein and the water removed by lyophilization. The reconstituted matrix is then packed in gelatin capsules and implanted subcutaneously

near the thigh muscles of young (one to two months old) rats. Varying amounts of protein preparations are used together with a constant amount of IBM in each capsule to determine the efficacy of the different protein preparations. Osteogenic activity induced by an implant is estimated by the examination of the excised implant (also referred to herein as an explant) by two approaches, (a) measuring the level of the enzyme alkaline phosphatase in the tissues developed at the implantation site, such tissue being excised at 17 to 20 days following implantation of a preparation for which osteogenic activity is to be determined and (b) performing a histologic examination of a 5 to 7 micron thick section of the tissue developed at the implant site following staining of paraffin-fixed sections of this tissue with toluidine blue (stains cartilage matrix), hematoxylin-eosin (resolves fibrous, cartilaginous and bone tissues) and von Kossa silver stain (for calcified matrix of bone tissue).

The level of alkaline phosphatase is measured since active bone formation is characteristically preceded by a significant surge of this enzyme and continued formation of bone is accompanied by a stable elevated level of alkaline phosphatase activity compared to that found in non-bone fibrous tissue surrounding the implants. An approximate quantitation of the levels of bone inducing activity in a protein preparation has been obtained by quantitating the level of alkaline phosphatase per unit weight of explant tissue. In practice, the explant tissue is homogenized in an appropriate buffer such as Tris-saline, dissociated with a nonionic detergent and the solubilized enzymes that are released from the tissue are obtained by removing the debris using centrifugation. The levels of alkaline phosphatase are quantitated by measuring the conversion of paranitrophenylphosphate to paranitrophenol catalyzed by dilutions of the test extract and calculating from a standard curve of known enzyme activity.

For bioassay studies, partially purified protein pools (i.e., alpha, beta, gamma I, gamma II, and delta as previously described herein) were obtained from Sephacryl S-200 column chromatography of a calf bone extract as previously described herein. These respective protein pools were reconstituted with IBM and implanted subcutaneously in rat thighs. Measurement of alkaline phosphatase activity and histological evaluation of sections of explants removed 17 to 20 days after implantation, showed that the P1 and the P5a—P5b proteins do not have bone inducing activity. The bioassay studies indicated the presence of maximum osteogenic activity in proteins in pools gamma I and gamma II. The three major components of the gamma fractions, that is, the P2, the P3 and the P4 proteins were purified to an essentially homogeneous state using reverse phase HPLC and described herein. The purified proteins, either singly or in a complete mixture, were reconstituted with inactive bone matrix and a bone induction assay performed. The results are shown in Table 6.

TABLE 6

| | Alkaline phosphatase (units/g) | Histology |
|---|---|---|
| IBM* Alone | <5 | Fibrous Tissue |
| IBM+750 μg P2 protein | <5 | Fibrous Tissue |
| IBM+750 μg P3 protein | 78 | New Bone |
| IBM+1000 μg P4 protein | <5 | Fibrous Tissue (a small trace of cartilage) |
| IBM+250 μg each of P2, P3 and P4 proteins | 63 | New Bone |

* "IBM" means Inactive Bone Matrix.
"<" means less than.

The data in Table 6 indicates that the calf P3 protein induced the formation of bone. Implants containing the calf P3 preparation developed into tissues that contained high levels of alkaline phosphatase enzyme activity. In contrast, implants prepared by reconstituting with either the P2 or the P4 preparation failed to produce detectable bone. When all three proteins were used in combination, significant bone formation was observed and high levels of alkaline phosphatase enzyme were obtained with one-third the amount of P3 protein (as compared to the P3 protein implant alone). It thus appears that at low concentrations of P3 protein, the presence of the P2 and/or the P4 protein provides enhancement of osteogenesis induced by the P3 protein.

In similar testing, purified P3 proteins obtained from human bone or porcine bone were reconstituted with IBM and implanted. Approximately three weeks later explants of tissue surrounding the implantation site were examined for alkaline phosphatase activity and histological characteristics. In each case, 750 μg of either P3 protein induced the formation of bone.

In using the active preparations described herein an osteogenic amount of one or more of the P3 proteins and/or osteogenically active polypeptides and/or immunologically related osteogenically active entities, with or without a pharmaceutically acceptable carrier, is administered at or in the proximity of the site in the mammal at which bone induction is desired. Administration will depend on the age, condition, sex and other characteristics of the subject to be treated. Preferred administration is by implantation, local injection or time controlled delivery using microcapsules, or other devices. Dosages will depend on the site and configuration of the area to be healed, such as, for example, a fracture zone. For example, a 5 cubic millimeter bone chip can be obtained with about 100 to 200 micrograms (μg) of P3 protein administered or implanted locally in the form of an implant in about 100 mg of IBM.

Active preparations can include other suitable bioactive materials such as growth factors, cell attachment factors, chemotactic agents, steroids, antibiotics, antiinflammatory agents and the like.

**Claims**

1. A method for isolating a preparation of an osteogenic protein which is a member of the P3 family of immunologically related proteins, said preparation being essentially homogeneous with respect to a molecular weight of from 22,000 daltons to 24,000 daltons, from dimineralized bone tissue comprising:

(a) treating said demineralized bone tissue under aqueous conditions with a solubilizing agent for said osteogenic protein and thereby extracting the osteogenic protein into solution with said solubilizing agent;

(b) subjecting said solution to a first size fractionation to recover fractions of proteins, identifying the fractions containing said osteogenic protein by measuring their bone inducing activity combining said fractions containing said osteogenic proteins to a first pool of proteins and concentrating said first pool;

(c) subjecting said concentrated first pool to a second size fractionation to recover fractions of proteins of a molecular weight between 12,000 daltons and 40,000 daltons, again identifying the fractions containing said osteogenic protein by measuring their bone inducing activity, combining said fractions containing said osteogenic protein to a second pool and concentrating said second pool;

(d) subjecting said concentrated second pool to a dialysis step comprising either (i) dialysis of said concentrated pool against an aqueous solvent containing trifluoroacetic acid and acetonitrile and removal of the insoluble material to obtain a soluble fraction or (ii) dialysis against deionized water to form an insoluble pellet and solubilization of said pellet with an aqueous solvent comprising trifluoroacetic acid and acetonitrile to obtain a soluble fraction;

(e) subjecting the soluble fraction of step (d) to reverse phase high performance liquid chromatography; and

(f) recovering a preparation of an osteogenic protein.

2. The method of Claim 1 wherein said solubilizing agent is guanidinium-hydrochloride.

3. The method of Claim 2 wherein the concentrated second pool of proteins of step (c) comprises proteins of molecular weight between 16,000 daltons and 25,000 daltons.

4. The method of Claim 3 wherein the size fractionations in steps (b) and (c) are conducted by gel filtration.

5. A preparation of an osteogenic protein which is a member of the P3 family of immunologically related proteins, said preparation being essentially homogeneous with respect to a molecular weight of from 22,000 daltons to 24,000 daltons and having the capacity, in such essentially homogeneous state, of promoting osteogenesis in a mammal, produced according to the process of Claim 1.

6. The preparation of Claim 5 which is isolated from bovine bone.

7. The preparation of Claim 5 which is isolated from human bone.

8. The preparation of Claim 5 which is isolated from porcine bone.

**Patentansprüche**

1. Verfahren zur Isolierung einer Präparation eines osteogenen Proteins, das ein Mitglied der P3-Familie von immunologisch verwandten Proteinen ist, wobei die Präparation im wesentlichen homogen im Hinblick auf ein Molekulargewicht von 22000 Dalton bis 24000 Dalton ist, aus entmineralisiertem Knochengewebe, umfassend:

(a) Behandeln des entmineralisierten Knochengewebes unter wäßrigen Bedingungen mit einem Solubilisierungsmittel für das osteogene Protein und dadurch Extrahieren des osteogenen Proteins in die Lösung mit dem Solubilisierungsmittel;

(b) Unterwerfen der Lösung einer ersten Größenfraktionierung, um Fraktionen von Proteinen zu erhalten, Identifizierung der Fraktionen, enthaltend das osteogene Protein durch Messung ihrer Knocheninduzierenden Aktivität, Kombinieren der Fraktionen, enthaltend die osteogenen Proteine zu einem ersten Pool von Proteinen und Konzentrieren des ersten Pools;

(c) Unterwerfen des konzentrierten ersten Pools einer zweiten Größenfraktionierung, um Fraktionen von Proteinen mit einem Molekulargewicht zwischen 12000 Dalton und 40000 Dalton zu erhalten, erneut Identifizieren der Fraktionen, enthaltend das osteogene Protein durch Messung ihrer Knocheninduzierenden Aktivität, Kombinieren der Fraktionen, enthaltend das osteogene Protein zu einem zweiten Pool und Konzentration des zweiten Pools;

(d) Unterwerfen des konzentrierten zweiten Pools einem Dialyseschritt, umfassend entweder (i) Dialyse des konzentrierten Pools gegen ein wäßriges Lösungsmittel, enthaltend Trifluoressigsäure und Acetonitril, und Entfernen des unlöslichen Materials, um eine lösliche Fraktion zu erhalten, oder (ii) Dialyse gegen entionisiertes Wasser, um ein unlösliches Pellet zu bilden und Solubilisierung des Pellets mit einem wäßrigen Lösungsmittel, umfassend Trifluoressigsäure und Acetonitril, um eine lösliche Fraktion zu erhalten;

(e) Unterwerfen der löslichen Fraktion von Schritt (d) einer Umkehrphasen-HPLC (high performance liquid)-Chromatographie; und

(f) Gewinnen der Präparation eines osteogenen Proteins.

2. Verfahren nach Anspruch 1, worin das Solubilisierungsmittel Guanidinhydrochlorid ist.

3. Verfahren nach Anspruch 2, worin der konzentrierte zweite Pool von Proteinen von Schritt (c) Proteine mit einem Molekulargewicht zwischen 16000 Dalton und 25000 Dalton umfaßt.

4. Verfahren nach Anspruch 3, worin die Größenfraktionierungen in den Schritten (b) und (c) durch Gelfiltration durchgeführt werden.

5. Präparation eines osteogenen Proteins, das ein Mitglied der P3-Familie von immunologisch verwandten Proteinen ist, wobei diese Präparation im wesentlichen homogen im Hinblick auf ein Molekulargewicht von zwischen 22000 Dalton und 24000 Dalton ist und die Fähigkeit besitzt in einen solchen, im wesentlichen homogenen Zustand, Osteogenese in einem Säugetier zu fördern, hergestellt gemäß dem Verfahren nach Anspruch 1.

6. Präparation nach Anspruch 5, die aus Rinderknochen isoliert ist.

7. Präparation nach Anspruch 5, die aus menschlichen Knochen isoliert ist.

8. Präparation nach Anspruch 5, die aus Schweineknochen isoliert ist.

**Revendications**

1. Procédé pour isoler une préparation d'une protéine ostéogénique appartenant à la famille P3 de protéines immunologiquement apparentées, ladite préparation étant essentiellement homogène relativement à un poids moléculaire de 22 000 daltons à 24 000 daltons, à partir de tissu osseux déminéralisé, consistant à:

(a) traiter ledit tissu osseux déminéralisé dans des conditions aqueuses avec un agent solubilisant de ladite protéine ostéogénique pour extraire la protéine ostéogénique en solution avec ledit agent solubilisant;

(b) soumettre ladite solution à un premier fractionnement par taille pour récupérer des fractions de protéines, identifier les fractions contenant ladite protéine ostéogénique par mesure de leur activité d'induction osseuse, combiner lesdites fractions contenant ladite protéine ostéogénique en un premier mélange de protéines et concentrer ledit premier mélange;

(c) soumettre ledit premier mélange concentré à un second fractionnement par taille pour récupérer les fractions de protéines ayant un poids moléculaire entre 12 000 daltons et 40 000 daltons, identifier à nouveau les fractions contenant ladite protéine ostéogénique par mesure de leur activité d'induction osseuse, combiner lesdites fractions contenant ladite protéine ostéogénique en un second mélange et concentrer ledit second mélange;

(d) soumettre ledit second mélange concentré à une étape de dialyse comprenant soit (i) la dialyse dudit mélange concentré contre un solvant aqueux contenant de l'acide trifluoroacétique et de l'acétonitrile et l'élimination de la matière insoluble pour obtenir une fraction soluble, ou (ii) la dialyse contre de l'eau désionisée pour former un culot insoluble et la solubilisation dudit culot avec un solvant aqueux comprenant de l'acide trifluoroacétique et de l'acétonitrile pour obtenir une fraction soluble;

(e) soumettre la fraction soluble du stade (d) à une chromatographie liquide haute performance en phase inverse; et

(f) récupérer une préparation d'une protéine ostéogénique.

2. Le procédé de la revendication 1, dans lequel ledit agent solubilisant est le chlorhydrate de guanidinium.

3. Le procédé de la revendication 2, dans lequel le second mélange concentré de protéines du stade (c) comprend des protéines de poids moléculaire entre 16 000 daltons et 25 000 daltons.

4. Le procédé de la revendication 3, dans lequel les fractionnements par taille des stades (b) et (c) sont effectués par filtration sur gel.

5. Une préparation d'une protéine ostéogénique qui appartient à la famille P3 de protéines immunologiquement apparentées, ladite préparation étant essentiellement homogène relativement à un poids moléculaire de 22 000 daltons à 24 000 daltons et ayant la capacité, sous cette forme essentiellement homogène, de faciliter l'ostéogenèse chez un mammifère, produite selon le procédé de la revendication 1.

6. La préparation de la revendication 5, qui est isolée d'os de bovins.

7. La préparation de la revendication 5, qui est isolée d'os humains.

8. La préparation de la revendication 5, qui est isolée d'os de porcins.

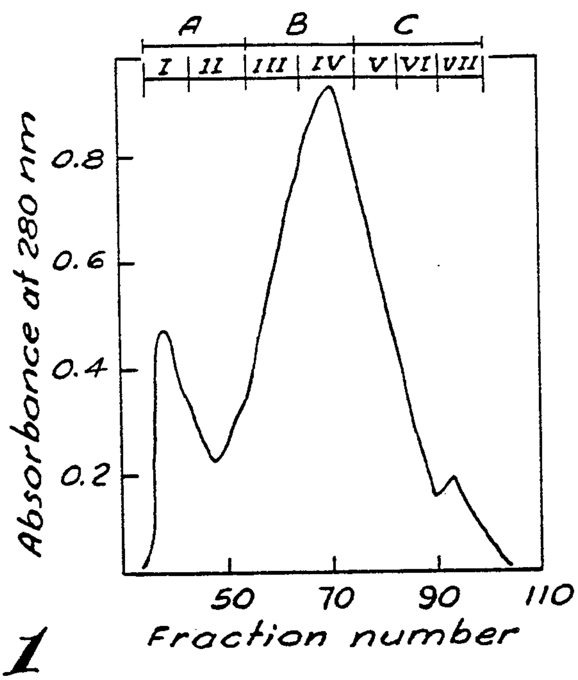
A
B
C
I
II
III
IV
V
VI
VII
0.8
0.6
0.4
0.2
Absorbance at 280 nm
50
70
90
110
Fraction number

Fig.1

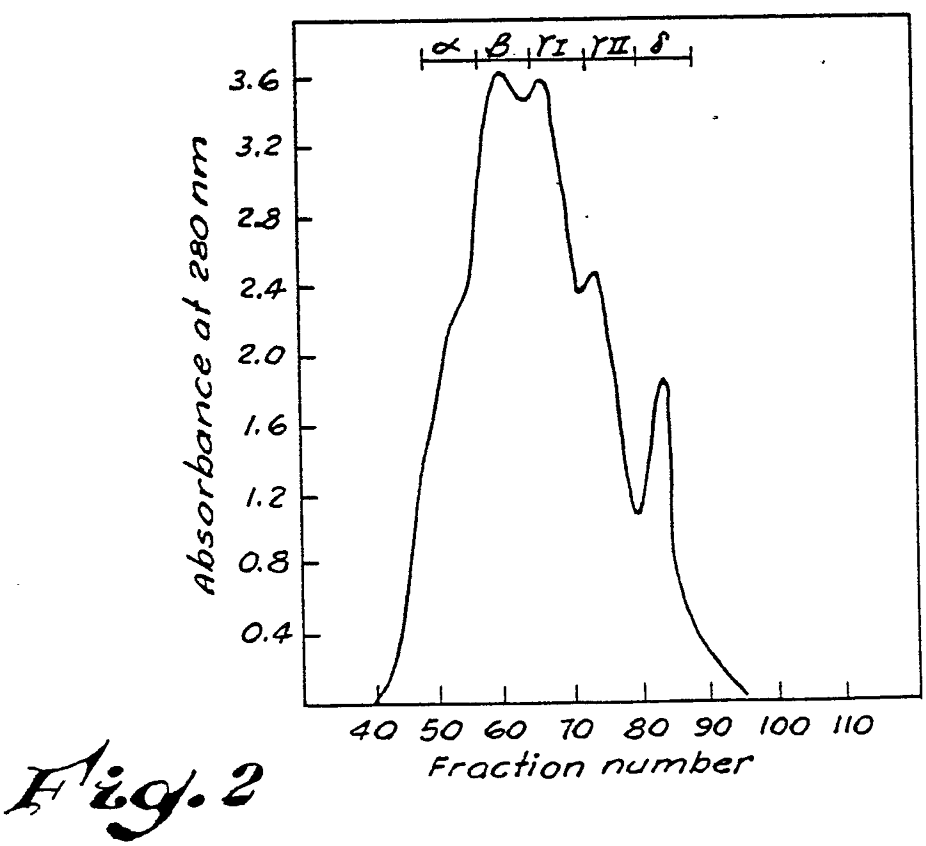
α
β
γI
γII
δ
3.6
3.2
2.8
2.4
2.0
1.6
1.2
0.8
0.4
Absorbance at 280 nm
40
50
60
70
80
90
100
110
Fraction number

Fig.2

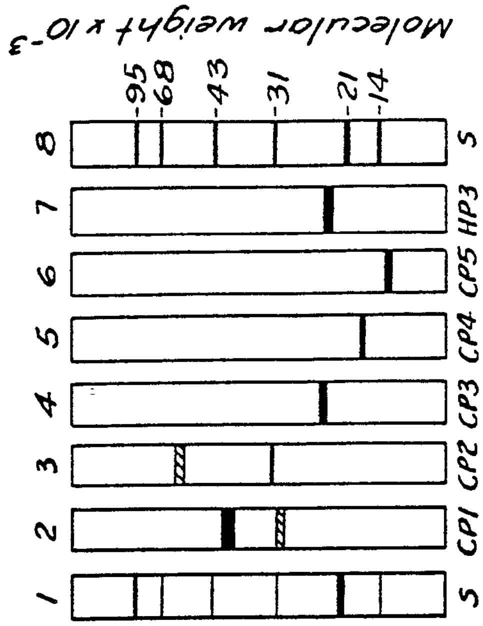

Molecular weight x 10^-3
-95
-68
-43
-31
-21
-14
1
2
3
4
5
6
7
8
S
CP1
CP2
CP3
CP4
CP5
HP3
S


Fig. 4

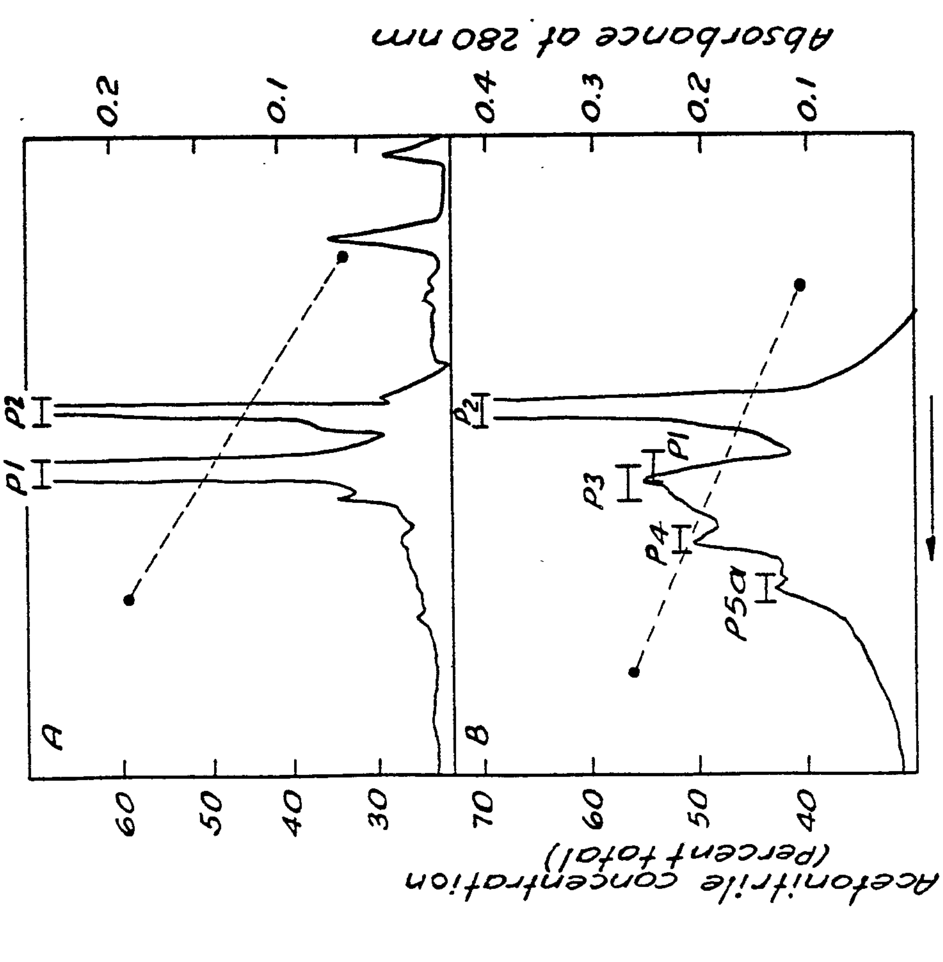

Absorbance at 280 nm
Acetonitrile concentration (percent total)
A
B
P1
P2
P3
P4
P5a
Increasing acetonitrile


Fig. 3

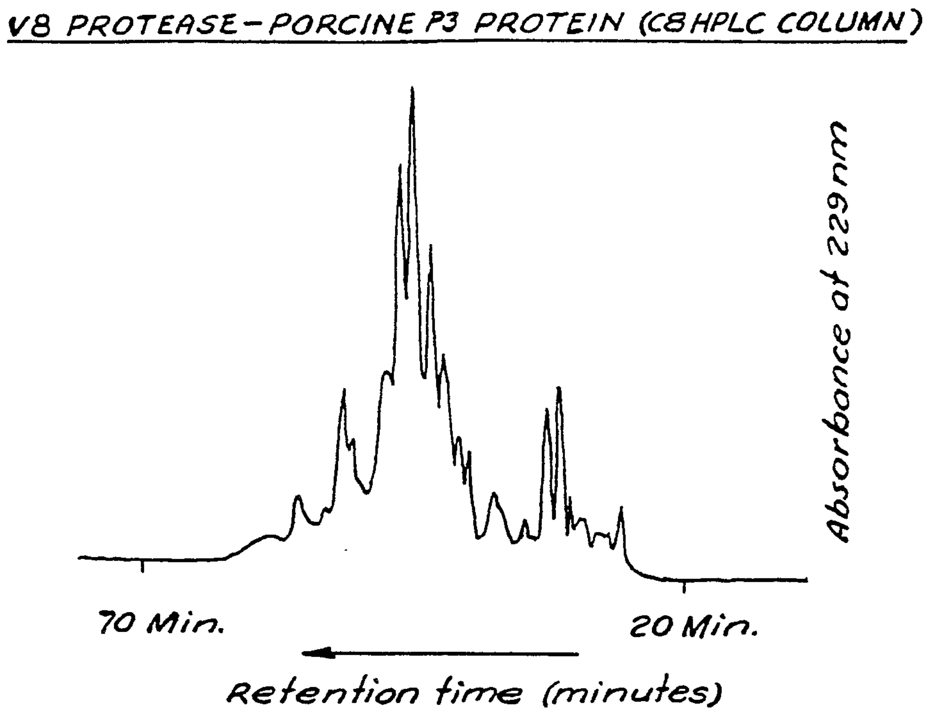
V8 PROTEASE-PORCINE P3 PROTEIN (C8 HPLC COLUMN)
Absorbance at 229nm
70 Min.
20 Min.
Retention time (minutes)

Fig. 5A

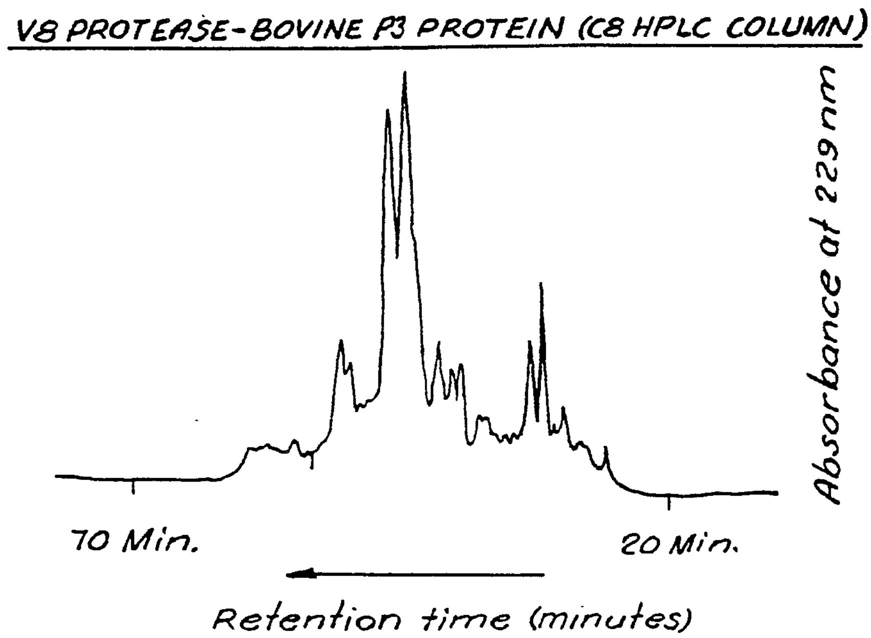
V8 PROTEASE-BOVINE P3 PROTEIN (C8 HPLC COLUMN)
Absorbance at 229nm
70 Min.
20 Min.
Retention time (minutes)

Fig. 5B

V8 PROTEASE - PORCINE P3 PROTEIN (C18 HPLC COLUMN)

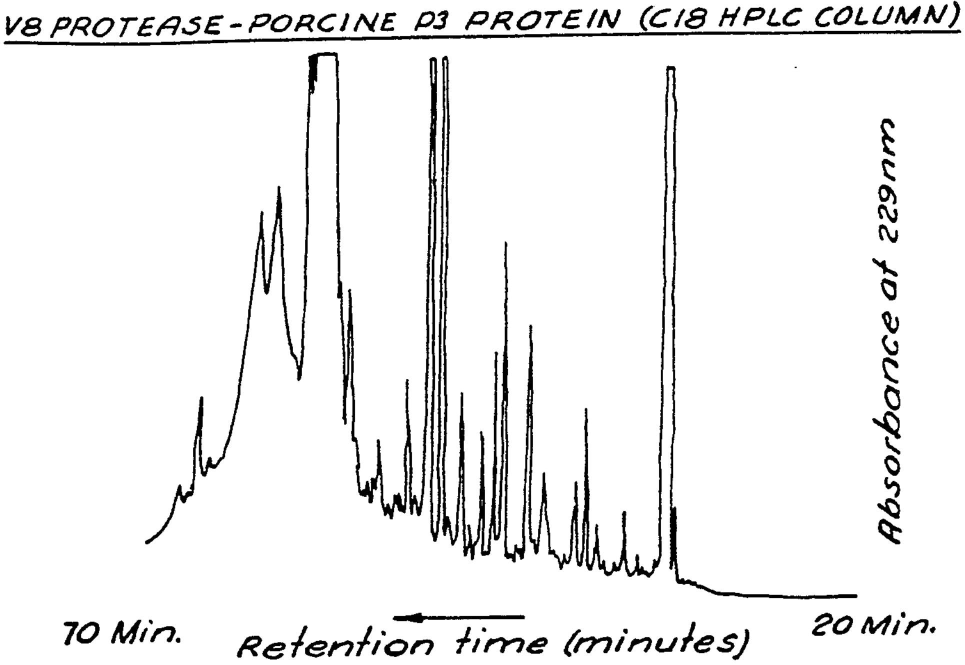


Fig. 6A

V8 PROTEASE - HUMAN P3 PROTEIN (C18 HPLC COLUMN)

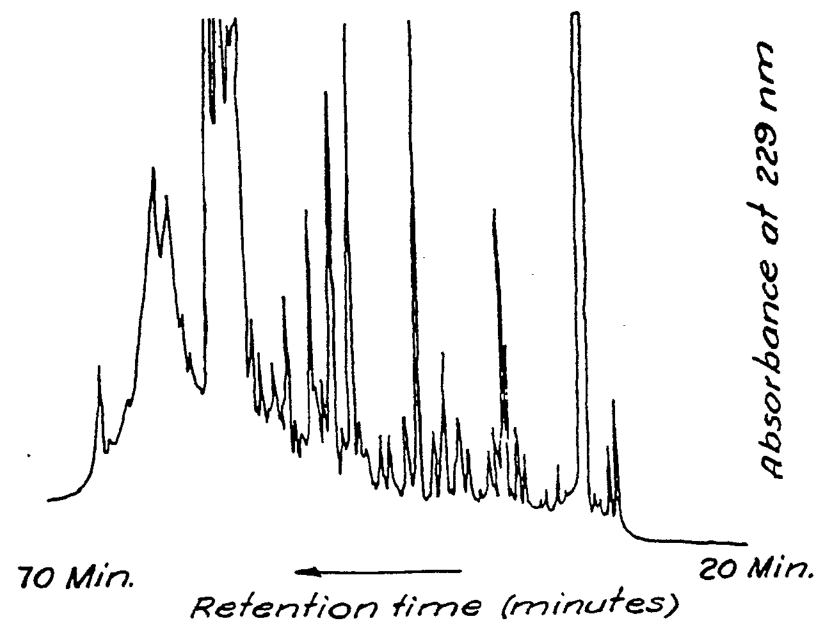


Fig. 6B

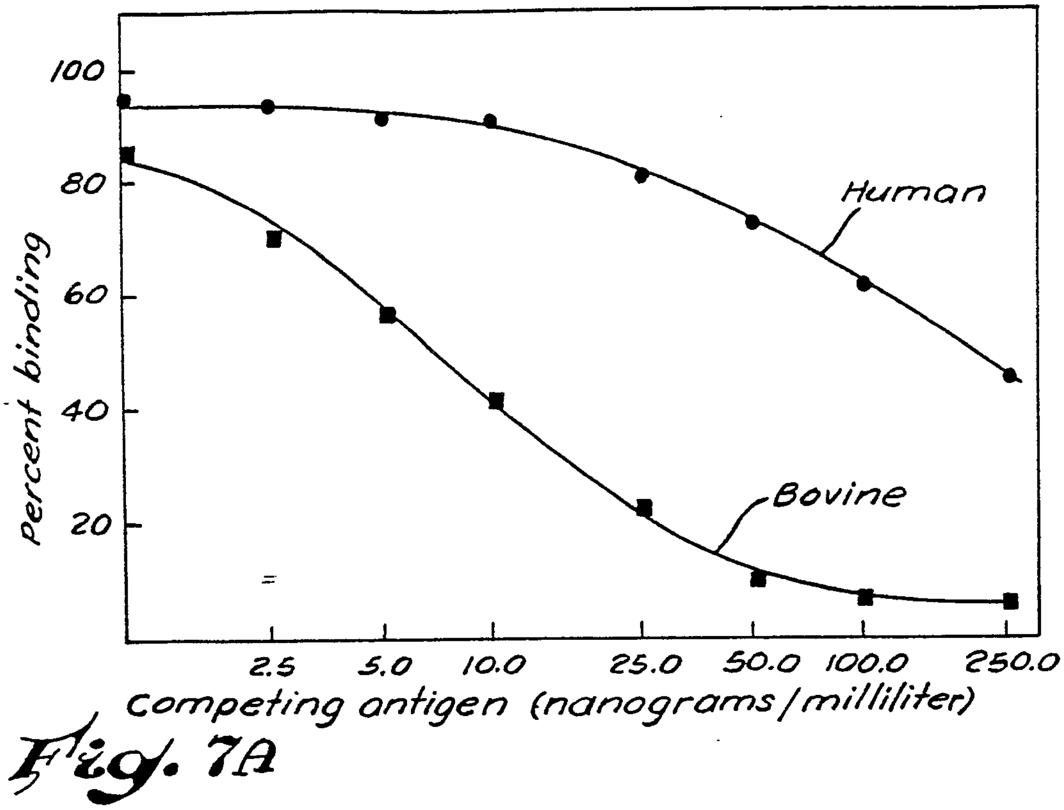
100
80
60
40
20
Percent binding
Human
Bovine
2.5
5.0
10.0
25.0
50.0
100.0
250.0
Competing antigen (nanograms/milliliter)

Fig. 7A

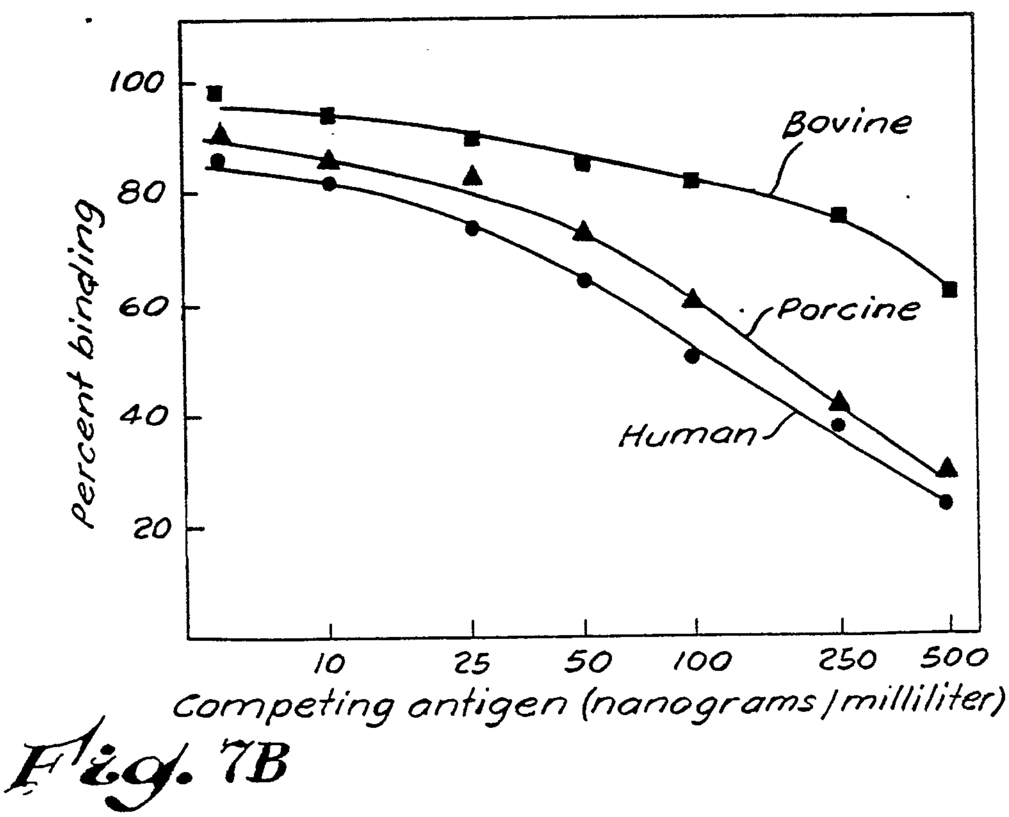
100
80
60
40
20
Percent binding
Bovine
Porcine
Human
10
25
50
100
250
500
Competing antigen (nanograms/milliliter)

Fig. 7B

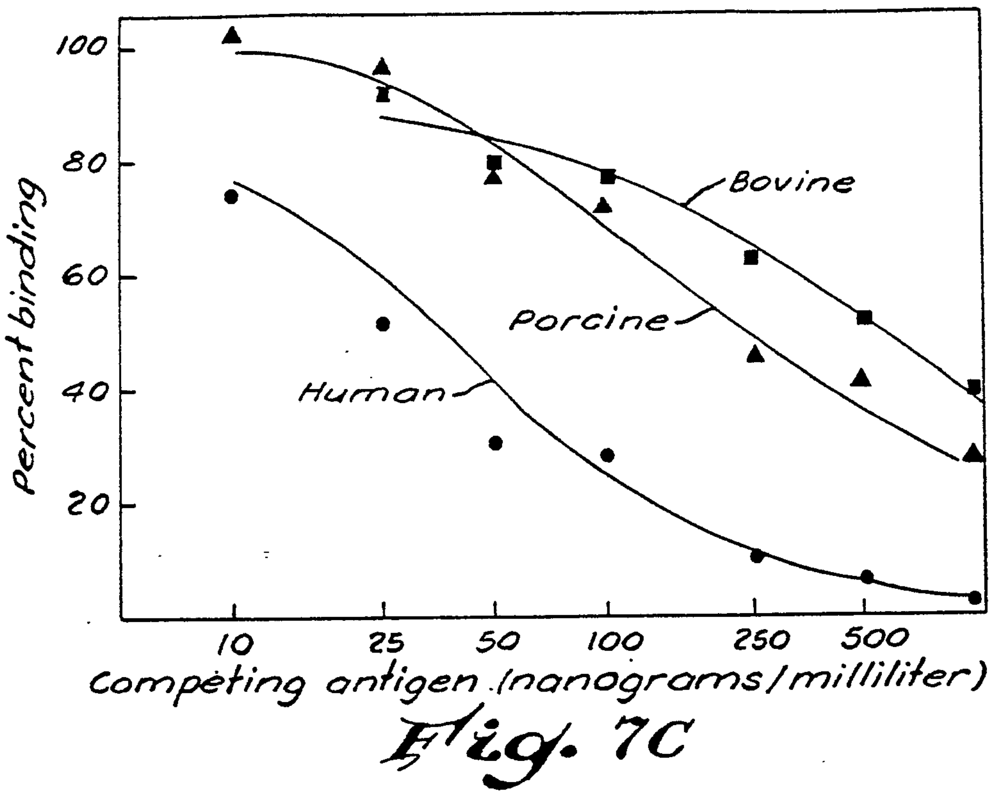
100
80
60
40
20
Percent binding
Bovine
Porcine
Human
10
25
50
100
250
500
Competing antigen (nanograms/milliliter)

Fig. 7C

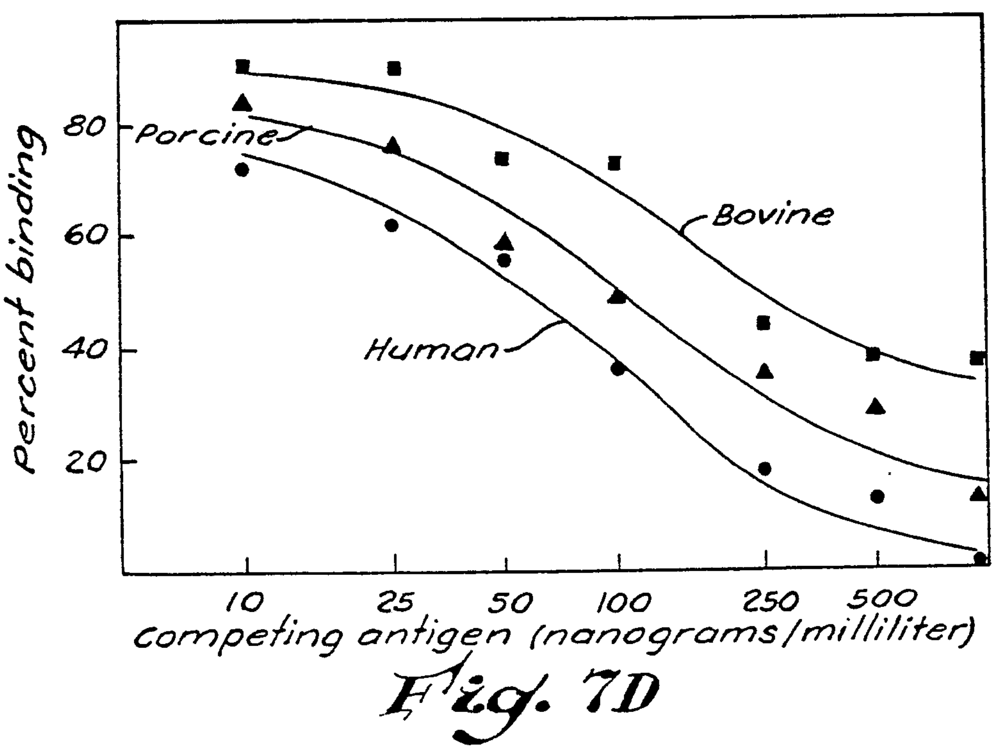
80
60
40
20
Percent binding
Porcine
Bovine
Human
10
25
50
100
250
500
Competing antigen (nanograms/milliliter)

Fig. 7D